# EUROPEAN PATENT APPLICATION

(11) **EP 4 772 179 A1**
(43) Date of publication of application: **08.07.2026**
(21) Application number: 24859947.4
(22) Date of filing: 30.08.2024
(51) Int. Cl.: A61K 31/519, A61K 31/337, A61K 31/357, A61K 31/475, A61K 31/506, A61K 31/513, A61K 31/4745, A61K 31/7048, A61K 31/7068, A61K 31/7072, A61K 31/7076, A61K 39/395, A61P 35/00

(54) **CANCER THERAPY INCLUDING 3,5-DISUBSTITUTED BENZENE ALKYNYL COMPOUND AND IMMUNE CHECKPOINT INHIBITOR**

(30) Priority: 31.08.2023 WO PCT/JP2023/032034; 05.04.2024 WO PCT/JP2024/014193
(71) Applicant: Taiho Pharmaceutical Co., Ltd., Chiyoda-ku, Tokyo 101-8444 (JP)
(72) Inventor: HIRAYAMA, Naoki, Tokyo 101-8444 (JP)
(74) Representative: Hoffmann Eitle
(86) International application number: PCT/JP2024/031131
(87) International publication number: WO 2025/047922

(57) **Abstract**

The problem to be solved by the present invention is to provide a novel combination therapy that has excellent antitumor effects. The present invention provides an antitumor agent (not comprising pembrolizumab as an active ingredient) comprising futibatinib and a salt thereof as active ingredients to be administered to a cancer patient in combination with an immune checkpoint inhibitor (except for a CD155/TIGIT pathway antagonist) and at least one or more other antitumor agents.

## Description

### Technical Field

The present invention relates to an antitumor agent, an antitumor effect enhancer, and a kit preparation.

### Background Art

Fibroblast growth factors (FGFs) are expressed in various tissues, and are one of the growth factors that regulate cell proliferation and differentiation. The physiological activity of the FGFs is mediated by fibroblast growth factor receptors (FGFRs), which are specific cell surface receptors. FGFRs belong to a receptor protein tyrosine kinase family, and comprise an extracellular ligand-binding domain, a single transmembrane domain, and an intracellular tyrosine kinase domain, and four types of FGFRs (FGFR1, FGFR2, FGFR3, and FGFR4) have been heretofore identified. FGFRs bind to FGFs to form dimers, and are activated by phosphorylation. Activation of the receptors induces mobilization and activation of specific downstream signal transduction molecules, thereby developing physiological functions. Some reports have been made about the relationship between aberrant FGF/FGFR signaling and various human tumors. Aberrant activation of FGF/FGFR signaling in human tumors is considered to be attributable to overexpression of FGFRs and/or gene amplification, gene mutation, chromosomal translocation, insertion, and inversion, gene fusion, or an autocrine or paracrine mechanism by overproduction of FGFs (ligands) (NPLs 1, 2, 3, and 4).

On the other hand, the development of cancer immunotherapy has progressed as a new cancer treatment method.

Activation of the adaptive immune response is initiated by the binding of antigenic peptide-MHC complexes to T-cell receptors (TCRs). This binding is further defined by costimulation or coinhibition by binding between the costimulatory molecule B7 family and their receptor CD28 family. That is, in order for T cells to activate an antigen specifically, two characteristic signal transduction events are required, and T cells that have received only antigen stimulation without costimulation from the B7 family enter a state of anergy, and immune tolerance is induced.

Taking advantage of this mechanism to suppress the activation of antigen-specific T cells, cancer cells escape from the immune surveillance system and continue to grow. Therefore, it is considered to be effective for cancer treatment to induce antitumor immune responses in the bodies of cancer patients by strengthening costimulation and blocking coinhibition, and to prevent tumor immune escape. Various proposals have been made for cancer immunotherapy targeting costimulatory molecules (stimulatory costimulatory molecules) or coinhibitory molecules (inhibitory costimulatory molecules) (NPL 5). For example, nivolumab (human IgG4 monoclonal antibody against human PD-1) is used for the treatment of malignant melanoma etc. as an immune checkpoint inhibitor that activates T cells by inhibiting the binding of PD-1 with its ligands (PD-L1 and PD-L2) (PTL 1 and NPL 6). Further, as a new immune checkpoint inhibitor, zimberelimab (AB122) is known, and is used for the treatment of Hodgkin lymphoma and the like (NPL 7).

(S)-1-(3-(4-amino-3-((3,5-dimethoxyphenyl)ethynyl)-1H-pyrazolo[3,4-d]pyrimidin-1-yl)pyrrolidin-1-yl)prop-2-en-1-one (hereinafter, referred to as "futibatinib"), which is a 3,5-disubstituted benzene alkynyl compound, or a salt thereof is known as an FGFR inhibitor, and combinations of FGFR inhibitors and other antitumor agents have been reported so far (PTL 2 and PTL 3). Further, a clinical trial (jRCT No.: jRCT2011210020) for combined administration of futibatinib and zimberelimab in patients with non-small cell lung cancer has been conducted (NPL 8).

### Citation List

### Patent Literatures

PTL 1: WO2004/004771
PTL 2: WO2013/108809
PTL 3: WO2017/150725
PTL 4: WO2016/161239

### Non-patent Literatures

NPL 1: Nat. Rev. Cancer 10: 116-129 (2010)
NPL 2: J. Clin. Oncol. 24, 3664-3671 (2006)
NPL 3: Mol. Cancer Res. 3, 655-667 (2005)
NPL 4: Cancer Res.70, 2085-2094 (2010)
NPL 5: Nat. Rev. Cancer, 12: 252-264 (2012)
NPL 6: N. Engl. J. Med., 366: 2443-2454 (2012)
NPL 7: Drugs. 81 (17): 2063-2068 (2021)
NPL 8: https://jrct.niph.go.jp/latest-detail/jRCT2011210020

### Summary of Invention

### Technical Problem

An object of the present invention is to provide a novel combination therapy that has excellent antitumor effects for cancer patients.

### Solution to Problem

In view of such circumstances, the present inventors found that the above problems can be solved by further using another antitumor agent in combination with the combined use of futibatinib or a salt thereof, and a specific immune checkpoint inhibitor.

Therefore, the present invention provides the following Items 1 to 55.

Item 1. The following antitumor agent according to Item 1-1, pharmaceutical composition according to Item 1-2, use according to Item 1-3, compound for use or a salt thereof according to Item 1-4, use according to Item 1-5, commercial package according to Item 1-6, or method according to Item 1-7:
Item 1-1. An antitumor agent of any one of the following (i) to (iii) (provided that an antitumor agent comprising pembrolizumab as an active ingredient and an antitumor agent used so as to be administered in combination with pembrolizumab are excluded):
   (i) the antitumor agent comprising, as an active ingredient, futibatinib or a pharmaceutically acceptable salt thereof, which is used so as to be administered to a cancer patient in combination with an immune checkpoint inhibitor and at least one or more other antitumor agents;
   (ii) the antitumor agent comprising, as an active ingredient, an immune checkpoint inhibitor, which is used so as to be administered to a cancer patient in combination with futibatinib or a pharmaceutically acceptable salt thereof and at least one or more other antitumor agents; and
   (iii) the antitumor agent comprising, as an active ingredient, at least one or more other antitumor agents, which is used so as to be administered to a cancer patient in combination with futibatinib or a pharmaceutically acceptable salt thereof and an immune checkpoint inhibitor;
Item 1-2. A pharmaceutical composition for preventing or treating cancer of any one of the following (i) to (iii) (provided that a pharmaceutical composition comprising pembrolizumab as an active ingredient and a pharmaceutical composition used so as to be administered in combination with pembrolizumab are excluded):
   (i) the pharmaceutical composition comprising, as an active ingredient, futibatinib or a pharmaceutically acceptable salt thereof and further comprising a pharmaceutical carrier, which is used so as to be administered to a cancer patient in combination with an immune checkpoint inhibitor and at least one or more other antitumor agents;
   (ii) the pharmaceutical composition comprising, as an active ingredient, an immune checkpoint inhibitor and further comprising a pharmaceutical carrier, which is used so as to be administered to a cancer patient in combination with futibatinib or a pharmaceutically acceptable salt thereof and at least one or more other antitumor agents; and
   (iii) the pharmaceutical composition comprising, as an active ingredient, at least one or more other antitumor agents and further comprising a pharmaceutical carrier, which is used so as to be administered to a cancer patient in combination with futibatinib or a pharmaceutically acceptable salt thereof and an immune checkpoint inhibitor;
Item 1-3. Use of any one of the following (i) to (iii) (provided that use for producing an antitumor agent comprising pembrolizumab as an active ingredient and use for producing an antitumor agent used so as to be administered in combination with pembrolizumab are excluded):
   (i) use of futibatinib or a pharmaceutically acceptable salt thereof, for producing an antitumor agent used so as to be administered to a cancer patient in combination with an immune checkpoint inhibitor and at least one or more other antitumor agents;
   (ii) use of an immune checkpoint inhibitor, for producing an antitumor agent used so as to be administered to a cancer patient in combination with futibatinib or a pharmaceutically acceptable salt thereof and at least one or more other antitumor agents; and
   (iii) use of at least one or more other antitumor agents, for producing an antitumor agent used so as to be administered to a cancer patient in combination with futibatinib or a pharmaceutically acceptable salt thereof and an immune checkpoint inhibitor;
Item 1-4. A compound for use or a salt thereof according to any one of the following (i) to (iii) (provided that a compound used so as to be administered in combination with pembrolizumab and pembrolizumab for use are excluded):
   (i) futibatinib or a pharmaceutically acceptable salt thereof for use in prevention or treatment of cancer, which is used so as to be administered to a cancer patient in combination with an immune checkpoint inhibitor and at least one or more other antitumor agents;
   (ii) an immune checkpoint inhibitor for use in prevention or treatment of cancer, which is used so as to be administered to a cancer patient in combination with futibatinib or a pharmaceutically acceptable salt thereof and at least one or more other antitumor agents; and
   (iii) at least one or more other antitumor agents for use in prevention or treatment of cancer, which are used so as to be administered to a cancer patient in combination with futibatinib or a pharmaceutically acceptable salt thereof and an immune checkpoint inhibitor;
Item 1-5. Use of any one of the following (i) to (iii) (provided that use of pembrolizumab and use of a compound used so as to be administered in combination with pembrolizumab are excluded):
   (i) use of futibatinib or a pharmaceutically acceptable salt thereof for preventing or treating cancer, which is used so as to be administered to a cancer patient in combination with an immune checkpoint inhibitor and at least one or more other antitumor agents;
   (ii) use of an immune checkpoint inhibitor for preventing or treating cancer, which is used so as to be administered to a cancer patient in combination with futibatinib or a pharmaceutically acceptable salt thereof and at least one or more other antitumor agents; and
   (iii) use of at least one or more other antitumor agents for preventing or treating cancer, which are used so as to be administered to a cancer patient in combination with futibatinib or a pharmaceutically acceptable salt thereof and an immune checkpoint inhibitor;
Item 1-6. A commercial package of any one of the following (i) to (iii) (provided that a commercial package used so as to be administered in combination with a pharmaceutical composition comprising pembrolizumab as an active ingredient and pembrolizumab is excluded):
   (i) the commercial package comprising futibatinib or a pharmaceutically acceptable salt thereof as an active ingredient, together with an instruction manual for use thereof for preventing or treating cancer in a subject, which is used so as to be administered to a cancer patient in combination with an immune checkpoint inhibitor and at least one or more other antitumor agents;
   (ii) the commercial package comprising an immune checkpoint inhibitor as an active ingredient, together with an instruction manual for use thereof for preventing or treating cancer in a subject, which is used so as to be administered to a cancer patient in combination with futibatinib or a pharmaceutically acceptable salt thereof, and at least one or more other antitumor agents; and
   (iii) the commercial package comprising an antitumor agent (other antitumor agent) other than "futibatinib or a pharmaceutically acceptable salt thereof and an immune checkpoint inhibitor" as an active ingredient, together with an instruction manual for use thereof for preventing or treating cancer in a subject, which is used so as to be administered to a cancer patient in combination with futibatinib or a pharmaceutically acceptable salt thereof and an immune checkpoint inhibitor.
Item 1-7. A method according to any one of the following (i) to (iii) (provided that a method comprising administering pembrolizumab and a method comprising administering an antitumor agent to a subject who has been administered, is administered simultaneously, or is to be administered pembrolizumab are excluded):
   (i) a method of preventing or treating cancer, comprising administering an effective amount of futibatinib or a pharmaceutically acceptable salt thereof to a subject in need thereof, in which the subject has been administered, is administered simultaneously, or is to be administered an immune checkpoint inhibitor, and the subject has been administered, is administered simultaneously, or is to be administered at least one or more other antitumor agents;
   (ii) a method of preventing or treating cancer, comprising administering an effective amount of an immune checkpoint inhibitor to a subject in need thereof, in which the subject has been administered, is administered simultaneously, or is to be administered futibatinib or a pharmaceutically acceptable salt thereof, and the subject has been administered, is administered simultaneously, or is to be administered at least one or more antitumor agents; and
   (ii) a method of preventing or treating cancer, comprising administering an effective amount of at least one or more antitumor agents (other antitumor agents) other than "futibatinib or a pharmaceutically acceptable salt thereof and an immune checkpoint inhibitor" to a subject in need thereof, in which the subject has been administered, is administered simultaneously, or is to be administered futibatinib or a pharmaceutically acceptable salt thereof, and the subject has been administered, is administered simultaneously, or is to be administered an immune checkpoint inhibitor.

Item 2. The antitumor agent, the pharmaceutical composition, the use, the compound for use or a salt thereof, the commercial package, or the method according to Item 1, wherein the immune checkpoint inhibitor is at least one selected from the group consisting of an anti-PD-1 antibody, an anti-PD-L1 antibody, and an anti-PD-L2 antibody.

Item 3. The antitumor agent, the pharmaceutical composition, the use, the compound for use or a salt thereof, the commercial package, or the method according to Item 1 or 2, wherein the immune checkpoint inhibitor is an anti-PD-1 antibody.

Item 4. The antitumor agent, the pharmaceutical composition, the use, the compound for use or a salt thereof, the commercial package, or the method according to Item 2 or 3, wherein the anti-PD-1 antibody is at least one selected from the group consisting of nivolumab, cemiplimab, spartalizumab, tislelizumab, BI754091, dostarlimab, sasanlimab, MGA-012, cetrelimab, AGEN-2034, zimberelimab, camrelizumab, budigalimab, and balstilimab.

Item 5. The antitumor agent, the pharmaceutical composition, the use, the compound for use or a salt thereof, the commercial package, or the method according to Item 4, wherein the anti-PD-1 antibody is zimberelimab.

Item 6. The antitumor agent, the pharmaceutical composition, the use, the compound for use or a salt thereof, the commercial package, or the method according to any one of Items 1 to 5, wherein the other antitumor agent is a chemotherapeutic agent.

Item 7. The antitumor agent, the pharmaceutical composition, the use, the compound for use or a salt thereof, the commercial package, or the method according to Item 6, wherein the chemotherapeutic agent is at least one selected from the group consisting of an antimetabolite, an alkaloid-based antitumor agent, a platinum preparation, an immune checkpoint inhibitor, a molecular targeting drug, an antitumor antibiotic, and an alkylating agent.

Item 8. The antitumor agent, the pharmaceutical composition, the use, the compound for use or a salt thereof, the commercial package, or the method according to Item 6, wherein the chemotherapeutic agent is at least one selected from the group consisting of an antimetabolite, an alkaloid-based antitumor agent, a platinum preparation, and an immune checkpoint inhibitor.

Item 9. The antitumor agent, the pharmaceutical composition, the use, the compound for use or a salt thereof, the commercial package, or the method according to Item 6, wherein the chemotherapeutic agent is at least one selected from the group consisting of fludarabine, cladribine, nelarabine, 5-fluorouracil, tegafur/gimeracil/oteracil potassium, tegafur/uracil, trifluridine/tipiracil hydrochloride, capecitabine, doxifluridine, 5-fluoro-2'-deoxyuridine, gemcitabine, cytarabine, pemetrexed, methotrexate, paclitaxel, albumin-bound paclitaxel, docetaxel, cabazitaxel, eribulin, irinotecan, nogitecan (topotecan), etoposide, teniposide, vinorelbine, vincristine, vinblastine, cisplatin, carboplatin, oxaliplatin, nedaplatin, domvanalimab, AB308, vibostolimab, ociperlimab, and tiragolumab.

Item 10. The antitumor agent, the pharmaceutical composition, the use, the compound for use or a salt thereof, the commercial package, or the method according to Item 6, wherein the chemotherapeutic agent is at least one selected from the group consisting of 5-fluorouracil, gemcitabine, albumin-bound paclitaxel, irinotecan, cisplatin, carboplatin, domvanalimab, and AB308.

Item 11. The antitumor agent, the pharmaceutical composition, the use, the compound for use or a salt thereof, the commercial package, or the method according to Item 6, wherein the chemotherapeutic agent is at least one selected from the group consisting of 5-fluorouracil, gemcitabine, albumin-bound paclitaxel, cisplatin, carboplatin, and domvanalimab.

Item 12. The antitumor agent, the pharmaceutical composition, the use, the compound for use or a salt thereof, the commercial package, or the method according to any one of Items 1 to 11, wherein the cancer is at least one selected from the group consisting of lung cancer, esophageal cancer, gastric cancer, duodenum cancer, liver cancer, hepatocellular cancer, biliary tract cancer, pancreatic cancer, large bowel cancer, breast cancer, uterine cancer, ovarian cancer, renal cancer, bladder cancer, prostate cancer, testicular tumor, thyroid cancer, bone or soft tissue tumor, leukemia, malignant lymphoma, multiple myeloma, head and neck cancer, brain tumor, mesothelioma, skin cancer, and cancer of unknown primary site.

Item 13. The antitumor agent, the pharmaceutical composition, the use, the compound for use or a salt thereof, the commercial package, or the method according to any one of Items 1 to 11, wherein the cancer is at least one selected from the group consisting of pancreatic cancer, lung cancer, esophageal cancer, biliary tract cancer, and head and neck cancer.

Item 14. The antitumor agent, the pharmaceutical composition, the use, the compound for use or a salt thereof, the commercial package, or the method according to any one of Items 1 to 13, wherein the cancer patient is a cancer patient who has not been previously treated for advanced cancer or who has been previously treated with one line of chemotherapy.

Item 15. The antitumor agent, the pharmaceutical composition, the use, the compound for use or a salt thereof, the commercial package, or the method according to any one of Items 1 to 14, wherein a 21-day or 28-day administration cycle is repeated once or twice or more.

Item 16. The antitumor agent, the pharmaceutical composition, the use, the compound for use or a salt thereof, the commercial package, or the method according to Item 15, wherein futibatinib is administered once a day on each day of the 21-day or 28-day administration cycle.

Item 17. The antitumor agent, the pharmaceutical composition, the use, the compound for use or a salt thereof, the commercial package, or the method according to Item 15, wherein zimberelimab is administered for one to two days in the 21-day or 28-day administration cycle.

Item 18. The antitumor agent, the pharmaceutical composition, the use, the compound for use or a salt thereof, the commercial package, or the method according to Item 15, wherein the other antitumor agent is administered for one to five days in the 21-day or 28-day administration cycle.

Item 19. The antitumor agent, the pharmaceutical composition, the use, the compound for use or a salt thereof, the commercial package, or the method according to Item 15, wherein the other antitumor agent is administered once on Day 1 in the 21-day or 28-day administration cycle.

Item 20. The antitumor agent, the pharmaceutical composition, the use, the compound for use or a salt thereof, the commercial package, or the method according to Item 15, wherein the other antitumor agent is administered once each on Day 1 and Day 8 in the 21-day or 28-day administration cycle.

Item 21. The antitumor agent, the pharmaceutical composition, the use, the compound for use or a salt thereof, the commercial package, or the method according to Item 15, wherein the other antitumor agent is administered once each on Day 1, Day 8, and Day 15 in the 21-day or 28-day administration cycle.

Item 22. The antitumor agent, the pharmaceutical composition, the use, the compound for use or a salt thereof, the commercial package, or the method according to Item 15, wherein the other antitumor agent is administered once each on Days 1, 2, 3, and 4 in the 21-day or 28-day administration cycle.

Item 23. The antitumor agent, the pharmaceutical composition, the use, the compound for use or a salt thereof, the commercial package, or the method according to Item 15, wherein the other antitumor agent is administered once each on Days 1, 2, 3, 4, and 5 in the 21-day or 28-day administration cycle.

Item 24. The antitumor agent, the pharmaceutical composition, the use, the compound for use or a salt thereof, the commercial package, or the method according to Item 16, wherein a dose of futibatinib is 8 mg/dose to 24 mg/dose.

Item 25. The antitumor agent, the pharmaceutical composition, the use, the compound for use or a salt thereof, the commercial package, or the method according to Item 16, wherein a dose of futibatinib is 12 mg/dose, 16 mg/dose, or 20 mg/dose.

Item 26. The antitumor agent, the pharmaceutical composition, the use, the compound for use or a salt thereof, the commercial package, or the method according to Item 16, wherein a dose of futibatinib is 20 mg/dose.

Item 27. The antitumor agent, the pharmaceutical composition, the use, the compound for use or a salt thereof, the commercial package, or the method according to Item 17, wherein a dose of zimberelimab is 240 mg/dose to 360 mg/dose.

Item 28. The antitumor agent, the pharmaceutical composition, the use, the compound for use or a salt thereof, the commercial package, or the method according to any one of Items 1 to 27, which is the following antitumor agent according to Item 28-1, pharmaceutical composition according to Item 28-2, use according to Item 28-3, compound for use or a salt thereof according to Item 28-4, use according to Item 28-5, commercial package according to Item 28-6, or method according to Item 28-7:
Item 28-1. An antitumor agent of any one of the following (i) to (iv) :
   (i) the antitumor agent comprising, as an active ingredient, futibatinib or a pharmaceutically acceptable salt thereof, which is used so as to be administered to a patient with esophageal cancer in combination with zimberelimab, cisplatin, and 5-fluorouracil;
   (ii) the antitumor agent comprising, as an active ingredient, zimberelimab, which is used so as to be administered to a patient with esophageal cancer in combination with futibatinib or a pharmaceutically acceptable salt thereof, cisplatin, and 5-fluorouracil;
   (iii) the antitumor agent comprising, as an active ingredient, cisplatin, which is used so as to be administered to a patient with esophageal cancer in combination with futibatinib or a pharmaceutically acceptable salt thereof, zimberelimab, and 5-fluorouracil; and
   (iv) the antitumor agent comprising, as an active ingredient, 5-fluorouracil, which is used so as to be administered to a patient with esophageal cancer in combination with futibatinib or a pharmaceutically acceptable salt thereof, zimberelimab, and cisplatin.
Item 28-2. A pharmaceutical composition of any one of the following (i) to (iv) (provided that a pharmaceutical composition comprising pembrolizumab as an active ingredient and a pharmaceutical composition used so as to be administered in combination with pembrolizumab are excluded):
   (i) the pharmaceutical composition comprising, as an active ingredient, futibatinib or a pharmaceutically acceptable salt thereof and further comprising a pharmaceutical carrier, which is used so as to be administered to a patient with esophageal cancer in combination with zimberelimab, cisplatin, and 5-fluorouracil;
   (ii) the pharmaceutical composition comprising, as an active ingredient, zimberelimab and further comprising a pharmaceutical carrier, which is used so as to be administered to a patient with esophageal cancer in combination with futibatinib or a pharmaceutically acceptable salt thereof, cisplatin, and 5-fluorouracil;
   (iii) the pharmaceutical composition comprising, as an active ingredient, cisplatin and further comprising a pharmaceutical carrier, which is used so as to be administered to a patient with esophageal cancer in combination with futibatinib or a pharmaceutically acceptable salt thereof, zimberelimab, and 5-fluorouracil; and
   (iv) the pharmaceutical composition comprising, as an active ingredient, 5-fluorouracil and further comprising a pharmaceutical carrier, which is used so as to be administered to a patient with esophageal cancer in combination with futibatinib or a pharmaceutically acceptable salt thereof, zimberelimab, and cisplatin.
Item 28-3. Use of any one of the following (i) to (iv) (provided that use for producing an antitumor agent comprising pembrolizumab as an active ingredient and use for producing an antitumor agent used so as to be administered in combination with pembrolizumab are excluded) :
   (i) use of futibatinib or a pharmaceutically acceptable salt thereof, for producing an antitumor agent used so as to be administered to a patient with esophageal cancer in combination with zimberelimab, cisplatin, and 5-fluorouracil;
   (ii) use of zimberelimab, for producing an antitumor agent used so as to be administered to a patient with esophageal cancer in combination with futibatinib or a pharmaceutically acceptable salt thereof, cisplatin, and 5-fluorouracil;
   (iii) use of cisplatin, for producing an antitumor agent used so as to be administered to a patient with esophageal cancer in combination with futibatinib or a pharmaceutically acceptable salt thereof, zimberelimab, and 5-fluorouracil; and
   (iv) use of 5-fluorouracil, for producing an antitumor agent used so as to be administered to a patient with esophageal cancer in combination with futibatinib or a pharmaceutically acceptable salt thereof, zimberelimab, and cisplatin.
Item 28-4. A compound for use or a salt thereof according to any one of the following (i) to (iv) (provided that a compound used so as to be administered in combination with pembrolizumab and pembrolizumab for use are excluded):
   (i) futibatinib or a pharmaceutically acceptable salt thereof for use in prevention or treatment of esophageal cancer, which is used so as to be administered to a patient with esophageal cancer in combination with zimberelimab, cisplatin, and 5-fluorouracil;
   (ii) zimberelimab for use in prevention or treatment of esophageal cancer, which is used so as to be administered to a patient with esophageal cancer in combination with futibatinib or a pharmaceutically acceptable salt thereof, cisplatin, and 5-fluorouracil;
   (iii) cisplatin for use in prevention or treatment of esophageal cancer, which is used so as to be administered to a patient with esophageal cancer in combination with futibatinib or a pharmaceutically acceptable salt thereof, zimberelimab, and 5-fluorouracil; and
   (iv) 5-fluorouracil for use in prevention or treatment of esophageal cancer, which is used so as to be administered to a patient with esophageal cancer in combination with futibatinib or a pharmaceutically acceptable salt thereof, zimberelimab, and cisplatin.
Item 28-5. Use of any one of the following (i) to (iv) (provided that use of pembrolizumab and use of a compound used so as to be administered in combination with pembrolizumab are excluded) :
   (i) use of futibatinib or a pharmaceutically acceptable salt thereof for preventing or treating esophageal cancer, which is used so as to be administered to a patient with esophageal cancer in combination with zimberelimab, cisplatin, and 5-fluorouracil;
   (ii) use of zimberelimab for preventing or treating esophageal cancer, which is used so as to be administered to a patient with esophageal cancer in combination with futibatinib or a pharmaceutically acceptable salt thereof, cisplatin, and 5-fluorouracil;
   (iii) use of cisplatin for preventing or treating esophageal cancer, which is used so as to be administered to a patient with esophageal cancer in combination with futibatinib or a pharmaceutically acceptable salt thereof, zimberelimab, and 5-fluorouracil; and
   (iv) use of 5-fluorouracil for preventing or treating esophageal cancer, which is used so as to be administered to a patient with esophageal cancer in combination with futibatinib or a pharmaceutically acceptable salt thereof, zimberelimab, and cisplatin.
Item 28-6. A commercial package of any one of the following (i) to (iv) (provided that a commercial package used so as to be administered in combination with a pharmaceutical composition comprising pembrolizumab as an active ingredient and pembrolizumab is excluded):
   (i) the commercial package comprising futibatinib or a pharmaceutically acceptable salt thereof as an active ingredient, together with an instruction manual for use thereof for preventing or treating esophageal cancer in a subject, which is used so as to be administered to a patient with esophageal cancer in combination with zimberelimab, cisplatin, and 5-fluorouracil;
   (ii) the commercial package comprising zimberelimab as an active ingredient, together with an instruction manual for use thereof for preventing or treating esophageal cancer in a subject, which is used so as to be administered to a patient with esophageal cancer in combination with futibatinib or a pharmaceutically acceptable salt thereof, cisplatin, and 5-fluorouracil;
   (iii) the commercial package comprising cisplatin as an active ingredient, together with an instruction manual for use thereof for preventing or treating esophageal cancer in a subject, which is used so as to be administered to a patient with esophageal cancer in combination with futibatinib or a pharmaceutically acceptable salt thereof, zimberelimab, and 5-fluorouracil; and
   (iv) the commercial package comprising 5-fluorouracil as an active ingredient, together with an instruction manual for use thereof for preventing or treating esophageal cancer in a subject, which is used so as to be administered to a patient with esophageal cancer in combination with futibatinib or a pharmaceutically acceptable salt thereof, zimberelimab, and cisplatin.
Item 28-7. A method according to any one of the following (i) to (iv) (provided that a method comprising administering pembrolizumab and a method comprising administering an antitumor agent to a subject who has been administered, is administered simultaneously, or is to be administered pembrolizumab are excluded):
   (i) a method of preventing or treating esophageal cancer, comprising administering an effective amount of futibatinib or a pharmaceutically acceptable salt thereof to a subject in need thereof, in which the subject has been administered, is administered simultaneously, or is to be administered zimberelimab, has been administered, is administered simultaneously, or is to be administered cisplatin, or has been administered, is administered simultaneously, or is to be administered 5-fluorouracil;
   (ii) a method of preventing or treating esophageal cancer, comprising administering an effective amount of zimberelimab to a subject in need thereof, in which the subject has been administered, is administered simultaneously, or is to be administered futibatinib or a pharmaceutically acceptable salt thereof, has been administered, is administered simultaneously, or is to be administered cisplatin, or has been administered, is administered simultaneously, or is to be administered 5-fluorouracil;
   (iii) a method of preventing or treating esophageal cancer, comprising administering an effective amount of cisplatin to a subject in need thereof, in which the subject has been administered, is administered simultaneously, or is to be administered futibatinib or a pharmaceutically acceptable salt thereof, has been administered, is administered simultaneously, or is to be administered zimberelimab, or has been administered, is administered simultaneously, or is to be administered 5-fluorouracil; and
   (iv) a method of preventing or treating esophageal cancer, comprising administering an effective amount of 5-fluorouracil to a subject in need thereof, in which the subject has been administered, is administered simultaneously, or is to be administered futibatinib or a pharmaceutically acceptable salt thereof, has been administered, is administered simultaneously, or is to be administered zimberelimab, or has been administered, is administered simultaneously, or is to be administered cisplatin.

Item 29. The antitumor agent, the pharmaceutical composition, the use, the compound for use or a salt thereof, the commercial package, or the method according to Item 28, wherein a 21-day administration cycle in which 12 mg/dose, 16 mg/dose, or 20 mg/dose of futibatinib is administered daily once a day, 360 mg/dose of zimberelimab is administered once, 80 mg/m²/dose of cisplatin is administered once, and 800 mg/m²/day of 5-fluorouracil is administered daily for 5 days is repeated once or twice or more.

Item 30. The antitumor agent, the pharmaceutical composition, the use, the compound for use or a salt thereof, the commercial package, or the method according to Item 28, wherein a 21-day administration cycle in which 12 mg/dose, 16 mg/dose, or 20 mg/dose of futibatinib is administered daily once a day, 360 mg/dose of zimberelimab is administered once on Day 1, 80 mg/m²/dose of cisplatin is administered once on Day 1, and 800 mg/m²/day of 5-fluorouracil is administered daily on Days 1 to 5 is repeated once or twice or more.

Item 31. The antitumor agent, the pharmaceutical composition, the use, the compound for use or a salt thereof, the commercial package, or the method according to any one of Items 28 to 30, wherein the cancer patient is a patient with esophageal cancer who has not been previously treated for advanced cancer.

Item 32. The antitumor agent, the pharmaceutical composition, the use, the compound for use or a salt thereof, the commercial package, or the method according to any one of Items 1 to 27, which is the following antitumor agent according to Item 32-1, pharmaceutical composition according to Item 32-2, use according to Item 32-3, compound for use or a salt thereof according to Item 32-4, use according to Item 32-5, commercial package according to Item 32-6, or method according to Item 32-7:
Item 32-1. An antitumor agent of any one of the following (i) to (iii) (provided that an antitumor agent comprising pembrolizumab as an active ingredient and an antitumor agent used so as to be administered in combination with pembrolizumab are excluded):
   (i) the antitumor agent comprising, as an active ingredient, futibatinib or a pharmaceutically acceptable salt thereof, which is used so as to be administered to a patient with esophageal cancer in combination with zimberelimab and domvanalimab;
   (ii) the antitumor agent comprising, as an active ingredient, zimberelimab, which is used so as to be administered to a patient with esophageal cancer in combination with futibatinib or a pharmaceutically acceptable salt thereof, and domvanalimab; and
   (iii) the antitumor agent comprising, as an active ingredient, domvanalimab, which is used so as to be administered to a patient with esophageal cancer in combination with futibatinib or a pharmaceutically acceptable salt thereof, and zimberelimab.
Item 32-2. A pharmaceutical composition for preventing or treating esophageal cancer of any one of the following (i) to (iii) (provided that a pharmaceutical composition comprising pembrolizumab as an active ingredient and a pharmaceutical composition used so as to be administered in combination with pembrolizumab are excluded):
   (i) the pharmaceutical composition comprising, as an active ingredient, futibatinib or a pharmaceutically acceptable salt thereof and further comprising a pharmaceutical carrier, which is used so as to be administered to a patient with esophageal cancer in combination with zimberelimab and domvanalimab;
   (ii) the pharmaceutical composition comprising, as an active ingredient, zimberelimab and further comprising a pharmaceutical carrier, which is used so as to be administered to a patient with esophageal cancer in combination with futibatinib or a pharmaceutically acceptable salt thereof, and domvanalimab; and
   (iii) the pharmaceutical composition comprising, as an active ingredient, domvanalimab and further comprising a pharmaceutical carrier, which is used so as to be administered to a patient with esophageal cancer in combination with futibatinib or a pharmaceutically acceptable salt thereof, and zimberelimab.
Item 32-3. Use of any one of the following (i) to (iii) (provided that use for producing an antitumor agent comprising pembrolizumab as an active ingredient and use for producing an antitumor agent used so as to be administered in combination with pembrolizumab are excluded):
   (i) use of futibatinib or a pharmaceutically acceptable salt thereof, for producing an antitumor agent used so as to be administered to a patient with esophageal cancer in combination with zimberelimab and domvanalimab;
   (ii) use of zimberelimab, for producing an antitumor agent used so as to be administered to a patient with esophageal cancer in combination with futibatinib or a pharmaceutically acceptable salt thereof, and domvanalimab; and
   (iii) use of domvanalimab, for producing an antitumor agent used so as to be administered to a patient with esophageal cancer in combination with futibatinib or a pharmaceutically acceptable salt thereof, and zimberelimab.
Item 32-4. A compound for use or a salt thereof according to any one of the following (i) to (iii) (provided that a compound used so as to be administered in combination with pembrolizumab and pembrolizumab for use are excluded):
   (i) futibatinib or a pharmaceutically acceptable salt thereof for use in prevention or treatment of esophageal cancer, which is used so as to be administered to a patient with esophageal cancer in combination with zimberelimab and domvanalimab;
   (ii) zimberelimab for use in prevention or treatment of esophageal cancer, which is used so as to be administered to a patient with esophageal cancer in combination with futibatinib or a pharmaceutically acceptable salt thereof, and domvanalimab; and
   (iii) domvanalimab for use in prevention or treatment of esophageal cancer, which is used so as to be administered to a patient with esophageal cancer in combination with futibatinib or a pharmaceutically acceptable salt thereof, and zimberelimab.
Item 32-5. Use of any one of the following (i) to (iii) (provided that use of pembrolizumab and use of a compound used so as to be administered in combination with pembrolizumab are excluded) :
   (i) use of futibatinib or a pharmaceutically acceptable salt thereof for preventing or treating esophageal cancer, which is used so as to be administered to a patient with esophageal cancer in combination with zimberelimab and domvanalimab;
   (ii) use of zimberelimab for preventing or treating esophageal cancer, which is used so as to be administered to a patient with esophageal cancer in combination with futibatinib or a pharmaceutically acceptable salt thereof, and domvanalimab; and
   (iii) use of domvanalimab for preventing or treating esophageal cancer, which is used so as to be administered to a patient with esophageal cancer in combination with futibatinib or a pharmaceutically acceptable salt thereof, and zimberelimab.
Item 32-6. A commercial package of any one of the following (i) to (iii) (provided that a commercial package used so as to be administered in combination with a pharmaceutical composition comprising pembrolizumab as an active ingredient and pembrolizumab is excluded):
   (i) the commercial package comprising futibatinib or a pharmaceutically acceptable salt thereof as an active ingredient, together with an instruction manual for use thereof for preventing or treating esophageal cancer in a subject, which is used so as to be administered to a patient with esophageal cancer in combination with zimberelimab and domvanalimab;
   (ii) the commercial package comprising zimberelimab as an active ingredient, together with an instruction manual for use thereof for preventing or treating esophageal cancer in a subject, which is used so as to be administered to a patient with esophageal cancer in combination with futibatinib or a pharmaceutically acceptable salt thereof, and domvanalimab; and
   (iii) the commercial package comprising domvanalimab as an active ingredient, together with an instruction manual for use thereof for preventing or treating esophageal cancer in a subject, which is used so as to be administered to a patient with esophageal cancer in combination with futibatinib or a pharmaceutically acceptable salt thereof, and zimberelimab.
Item 32-7. A method according to any one of the following (i) to (iii) (provided that a method comprising administering pembrolizumab and a method comprising administering an antitumor agent to a subject who has been administered, is administered simultaneously, or is to be administered pembrolizumab are excluded):
   (i) a method of preventing or treating esophageal cancer, comprising administering an effective amount of futibatinib or a pharmaceutically acceptable salt thereof to a subject in need thereof, in which the subject has been administered, is administered simultaneously, or is to be administered zimberelimab, and has been administered, is administered simultaneously, or is to be administered domvanalimab;
   (ii) a method of preventing or treating esophageal cancer, comprising administering an effective amount of zimberelimab to a subject in need thereof, in which the subject has been administered, is administered simultaneously, or is to be administered futibatinib or a pharmaceutically acceptable salt thereof, and has been administered, is administered simultaneously, or is to be administered domvanalimab; and
   (iii) a method of preventing or treating esophageal cancer, comprising administering an effective amount of domvanalimab to a subject in need thereof, in which the subject has been administered, is administered simultaneously, or is to be administered futibatinib or a pharmaceutically acceptable salt thereof, and has been administered, is administered simultaneously, or is to be administered zimberelimab.

Item 33. The antitumor agent, the pharmaceutical composition, the use, the compound for use or a salt thereof, the commercial package, or the method according to Item 32, wherein a 21-day administration cycle in which 12 mg/dose, 16 mg/dose, or 20 mg/dose of futibatinib is administered daily once a day, 360 mg/dose of zimberelimab is administered once, and 1200 mg/dose of domvanalimab is administered once is repeated once or twice or more.

Item 34. The antitumor agent, the pharmaceutical composition, the use, the compound for use or a salt thereof, the commercial package, or the method according to Item 32, wherein a 21-day administration cycle in which 12 mg/dose, 16 mg/dose, or 20 mg/dose of futibatinib is administered daily once a day, 360 mg/dose of zimberelimab is administered once on Day 1, and 1200 mg/dose of domvanalimab is administered once on Day 1 is repeated once or twice or more.

Item 35. The antitumor agent, the pharmaceutical composition, the use, the compound for use or a salt thereof, the commercial package, or the method according to any one of Items 32 to 34, wherein the cancer patient is a patient with esophageal cancer who has not been previously treated for advanced cancer or who has been previously treated with one line of chemotherapy.

Item 36. The antitumor agent, the pharmaceutical composition, the use, the compound for use or a salt thereof, the commercial package, or the method according to any one of Items 1 to 27, which is the following antitumor agent according to Item 36-1, pharmaceutical composition according to Item 36-2, use according to Item 36-3, compound for use or a salt thereof according to Item 36-4, use according to Item 36-5, commercial package according to Item 36-6, or method according to Item 36-7:
Item 36-1. An antitumor agent of any one of the following (i) to (iv) (provided that an antitumor agent comprising pembrolizumab as an active ingredient and an antitumor agent used so as to be administered in combination with pembrolizumab are excluded):
   (i) the antitumor agent comprising, as an active ingredient, futibatinib or a pharmaceutically acceptable salt thereof, which is used so as to be administered to a patient with head and neck cancer in combination with zimberelimab, 5-fluorouracil, and carboplatin or cisplatin;
   (ii) the antitumor agent comprising, as an active ingredient, zimberelimab, which is used so as to be administered to a patient with head and neck cancer in combination with futibatinib or a pharmaceutically acceptable salt thereof, 5-fluorouracil, and carboplatin or cisplatin;
   (iii) the antitumor agent comprising, as an active ingredient, 5-fluorouracil, which is used so as to be administered to a patient with head and neck cancer in combination with futibatinib or a pharmaceutically acceptable salt thereof, zimberelimab, and carboplatin or cisplatin; and
   (iv) the antitumor agent comprising, as an active ingredient, carboplatin or cisplatin, which is used so as to be administered to a patient with head and neck cancer in combination with futibatinib or a pharmaceutically acceptable salt thereof, zimberelimab, and 5-fluorouracil.
Item 36-2. A pharmaceutical composition for preventing or treating head and neck cancer of any one of the following (i) to (iv) (provided that a pharmaceutical composition comprising pembrolizumab as an active ingredient and a pharmaceutical composition used so as to be administered in combination with pembrolizumab are excluded):
   (i) the pharmaceutical composition comprising, as an active ingredient, futibatinib or a pharmaceutically acceptable salt thereof and further comprising a pharmaceutical carrier, which is used so as to be administered to a patient with head and neck cancer in combination with zimberelimab, 5-fluorouracil, and carboplatin or cisplatin;
   (ii) the pharmaceutical composition comprising, as an active ingredient, zimberelimab and further comprising a pharmaceutical carrier, which is used so as to be administered to a patient with head and neck cancer in combination with futibatinib or a pharmaceutically acceptable salt thereof, 5-fluorouracil, and carboplatin or cisplatin;
   (iii) the pharmaceutical composition comprising, as an active ingredient, 5-fluorouracil and further comprising a pharmaceutical carrier, which is used so as to be administered to a patient with head and neck cancer in combination with futibatinib or a pharmaceutically acceptable salt thereof, zimberelimab, and carboplatin or cisplatin; and
   (iv) the pharmaceutical composition comprising, as an active ingredient, carboplatin or cisplatin and further comprising a pharmaceutical carrier, which is used so as to be administered to a patient with head and neck cancer in combination with futibatinib or a pharmaceutically acceptable salt thereof, zimberelimab, and 5-fluorouracil.
Item 36-3. Use of any one of the following (i) to (iv) (provided that use for producing an antitumor agent comprising pembrolizumab as an active ingredient and use for producing an antitumor agent used so as to be administered in combination with pembrolizumab are excluded):
   (i) use of futibatinib or a pharmaceutically acceptable salt thereof, for producing an antitumor agent used so as to be administered to a patient with head and neck cancer in combination with zimberelimab, 5-fluorouracil, and carboplatin or cisplatin;
   (ii) use of zimberelimab, for producing an antitumor agent used so as to be administered to a patient with head and neck cancer in combination with futibatinib or a pharmaceutically acceptable salt thereof, 5-fluorouracil, and carboplatin or cisplatin;
   (iii) use of 5-fluorouracil, for producing an antitumor agent used so as to be administered to a patient with head and neck cancer in combination with futibatinib or a pharmaceutically acceptable salt thereof, zimberelimab, and carboplatin or cisplatin; and
   (iv) use of carboplatin or cisplatin, for producing an antitumor agent used so as to be administered to a patient with head and neck cancer in combination with futibatinib or a pharmaceutically acceptable salt thereof, zimberelimab, and 5-fluorouracil.
Item 36-4. A compound for use or a salt thereof according to any one of the following (i) to (iv) (provided that a compound used so as to be administered in combination with pembrolizumab and pembrolizumab for use are excluded):
   (i) futibatinib or a pharmaceutically acceptable salt thereof for use in prevention or treatment of head and neck cancer, which is used so as to be administered to a patient with head and neck cancer in combination with zimberelimab, 5-fluorouracil, and carboplatin or cisplatin;
   (ii) zimberelimab for use in prevention or treatment of head and neck cancer, which is used so as to be administered to a patient with head and neck cancer in combination with futibatinib or a pharmaceutically acceptable salt thereof, 5-fluorouracil, and carboplatin or cisplatin;
   (iii) 5-fluorouracil for use in prevention or treatment of head and neck cancer, which is used so as to be administered to a patient with head and neck cancer in combination with futibatinib or a pharmaceutically acceptable salt thereof, zimberelimab, and carboplatin or cisplatin; and
   (iv) carboplatin or cisplatin for use in prevention or treatment of head and neck cancer, which is used so as to be administered to a patient with head and neck cancer in combination with futibatinib or a pharmaceutically acceptable salt thereof, zimberelimab, and 5-fluorouracil.
Item 36-5. Use of any one of the following (i) to (iv) (provided that use of pembrolizumab and use of a compound used so as to be administered in combination with pembrolizumab are excluded) :
   (i) use of futibatinib or a pharmaceutically acceptable salt thereof for preventing or treating head and neck cancer, which is used so as to be administered to a patient with head and neck cancer in combination with zimberelimab, 5-fluorouracil, and carboplatin or cisplatin;
   (ii) use of zimberelimab for preventing or treating head and neck cancer, which is used so as to be administered to a patient with head and neck cancer in combination with futibatinib or a pharmaceutically acceptable salt thereof, 5-fluorouracil, and carboplatin or cisplatin;
   (iii) use of 5-fluorouracil for preventing or treating head and neck cancer, which is used so as to be administered to a patient with head and neck cancer in combination with futibatinib or a pharmaceutically acceptable salt thereof, zimberelimab, and carboplatin or cisplatin; and
   (iv) use of carboplatin or cisplatin for preventing or treating head and neck cancer, which is used so as to be administered to a patient with head and neck cancer in combination with futibatinib or a pharmaceutically acceptable salt thereof, zimberelimab, and 5-fluorouracil.
Item 36-6. A commercial package of any one of the following (i) to (iv) (provided that a commercial package used so as to be administered in combination with a pharmaceutical composition comprising pembrolizumab as an active ingredient and pembrolizumab is excluded):
   (i) the commercial package comprising futibatinib or a pharmaceutically acceptable salt thereof as an active ingredient, together with an instruction manual for use thereof for preventing or treating head and neck cancer in a subject, which is used so as to be administered to a patient with head and neck cancer in combination with zimberelimab, 5-fluorouracil, and carboplatin or cisplatin;
   (ii) the commercial package comprising zimberelimab as an active ingredient, together with an instruction manual for use thereof for preventing or treating head and neck cancer in a subject, which is used so as to be administered to a patient with head and neck cancer in combination with futibatinib or a pharmaceutically acceptable salt thereof, 5-fluorouracil, and carboplatin or cisplatin;
   (iii) the commercial package comprising 5-fluorouracil as an active ingredient, together with an instruction manual for use thereof for preventing or treating head and neck cancer in a subject, which is used so as to be administered to a patient with head and neck cancer in combination with futibatinib or a pharmaceutically acceptable salt thereof, zimberelimab, and carboplatin or cisplatin; and
   (iv) the commercial package comprising carboplatin or cisplatin as an active ingredient, together with an instruction manual for use thereof for preventing or treating head and neck cancer in a subject, which is used so as to be administered to a patient with head and neck cancer in combination with futibatinib or a pharmaceutically acceptable salt thereof, zimberelimab, and 5-fluorouracil.
Item 36-7. A method according to any one of the following (i) to (iv) (provided that a method comprising administering pembrolizumab and a method comprising administering an antitumor agent to a subject who has been administered, is administered simultaneously, or is to be administered pembrolizumab are excluded):
   (i) a method of preventing or treating head and neck cancer, comprising administering an effective amount of futibatinib or a pharmaceutically acceptable salt thereof to a subject in need thereof, in which the subject has been administered, is administered simultaneously, or is to be administered zimberelimab, has been administered, is administered simultaneously, or is to be administered 5-fluorouracil, or has been administered, is administered simultaneously, or is to be administered carboplatin or cisplatin;
   (ii) a method of preventing or treating head and neck cancer, comprising administering an effective amount of zimberelimab to a subject in need thereof, in which the subject has been administered, is administered simultaneously, or is to be administered futibatinib or a pharmaceutically acceptable salt thereof, has been administered, is administered simultaneously, or is to be administered 5-fluorouracil, or has been administered, is administered simultaneously, or is to be administered carboplatin or cisplatin;
   (iii) a method of preventing or treating head and neck cancer, comprising administering an effective amount of 5-fluorouracil to a subject in need thereof, in which the subject has been administered, is administered simultaneously, or is to be administered futibatinib or a pharmaceutically acceptable salt thereof, has been administered, is administered simultaneously, or is to be administered zimberelimab, or has been administered, is administered simultaneously, or is to be administered carboplatin or cisplatin; and
   (iv) a method of preventing or treating head and neck cancer, comprising administering an effective amount of carboplatin or cisplatin to a subject in need thereof, in which the subject has been administered, is administered simultaneously, or is to be administered futibatinib or a pharmaceutically acceptable salt thereof, has been administered, is administered simultaneously, or is to be administered zimberelimab, or has been administered, is administered simultaneously, or is to be administered 5-fluorouracil.

Item 37. The antitumor agent, the pharmaceutical composition, the use, the compound for use or a salt thereof, the commercial package, or the method according to Item 36, wherein a 21-day administration cycle in which 12 mg/dose, 16 mg/dose, or 20 mg/dose of futibatinib is administered daily once a day, 360 mg/dose of zimberelimab is administered once, AUC 5 carboplatin or 100 mg/m²/dose of cisplatin is administered once, and 1000 mg/m²/day of 5-fluorouracil is administered daily for 4 days is repeated once or twice or more.

Item 38. The antitumor agent, the pharmaceutical composition, the use, the compound for use or a salt thereof, the commercial package, or the method according to Item 36, wherein a 21-day administration cycle in which 12 mg/dose, 16 mg/dose, or 20 mg/dose of futibatinib is administered daily once a day, 360 mg/dose of zimberelimab is administered once on Day 1, AUC 5 carboplatin or 100 mg/m²/dose of cisplatin is administered once on Day 1, and 1000 mg/m²/day of 5-fluorouracil is administered daily on Days 1 to 4 is repeated once or twice or more.

Item 39. The antitumor agent, the pharmaceutical composition, the use, the compound for use or a salt thereof, the commercial package, or the method according to any one of Items 37 to 38, wherein the cancer patient is a patient with head and neck cancer who has not been previously treated for advanced cancer.

Item 40. The antitumor agent, the pharmaceutical composition, the use, the compound for use or a salt thereof, the commercial package, or the method according to any one of Items 1 to 27, which is the following antitumor agent according to Item 40-1, pharmaceutical composition according to Item 40-2, use according to Item 40-3, compound for use or a salt thereof according to Item 40-4, use according to Item 40-5, commercial package according to Item 40-6, or method according to Item 40-7:
Item 40-1. An antitumor agent of any one of the following (i) to (iii) (provided that an antitumor agent comprising pembrolizumab as an active ingredient and an antitumor agent used so as to be administered in combination with pembrolizumab are excluded):
   (i) the antitumor agent comprising, as an active ingredient, futibatinib or a pharmaceutically acceptable salt thereof, which is used so as to be administered to a patient with head and neck cancer in combination with zimberelimab and domvanalimab;
   (ii) the antitumor agent comprising, as an active ingredient, zimberelimab, which is used so as to be administered to a patient with head and neck cancer in combination with futibatinib or a pharmaceutically acceptable salt thereof, and domvanalimab; and
   (iii) the antitumor agent comprising, as an active ingredient, domvanalimab, which is used so as to be administered to a patient with head and neck cancer in combination with futibatinib or a pharmaceutically acceptable salt thereof, and zimberelimab.
Item 40-2. A pharmaceutical composition for preventing or treating head and neck cancer of any one of the following (i) to (iii) (provided that a pharmaceutical composition comprising pembrolizumab as an active ingredient and a pharmaceutical composition used so as to be administered in combination with pembrolizumab are excluded):
   (i) the pharmaceutical composition comprising, as an active ingredient, futibatinib or a pharmaceutically acceptable salt thereof and further comprising a pharmaceutical carrier, which is used so as to be administered to a patient with head and neck cancer in combination with zimberelimab and domvanalimab;
   (ii) the pharmaceutical composition comprising, as an active ingredient, zimberelimab and further comprising a pharmaceutical carrier, which is used so as to be administered to a patient with head and neck cancer in combination with futibatinib or a pharmaceutically acceptable salt thereof, and domvanalimab; and
   (iii) the pharmaceutical composition comprising, as an active ingredient, domvanalimab and further comprising a pharmaceutical carrier, which is used so as to be administered to a patient with head and neck cancer in combination with futibatinib or a pharmaceutically acceptable salt thereof, and zimberelimab.
Item 40-3. Use of any one of the following (i) to (iii) (provided that use for producing an antitumor agent comprising pembrolizumab as an active ingredient and use for producing an antitumor agent used so as to be administered in combination with pembrolizumab are excluded) :
   (i) use of futibatinib or a pharmaceutically acceptable salt thereof, for producing an antitumor agent used so as to be administered to a patient with head and neck cancer in combination with zimberelimab and domvanalimab;
   (ii) use of zimberelimab, for producing an antitumor agent used so as to be administered to a patient with head and neck cancer in combination with futibatinib or a pharmaceutically acceptable salt thereof, and domvanalimab; and
   (iii) use of domvanalimab, for producing an antitumor agent used so as to be administered to a patient with head and neck cancer in combination with futibatinib or a pharmaceutically acceptable salt thereof, and zimberelimab.
Item 40-4. A compound for use or a salt thereof according to any one of the following (i) to (iii) (provided that a compound used so as to be administered in combination with pembrolizumab and pembrolizumab for use are excluded):
   (i) futibatinib or a pharmaceutically acceptable salt thereof for use in prevention or treatment of head and neck cancer, which is used so as to be administered to a patient with head and neck cancer in combination with zimberelimab and domvanalimab;
   (ii) zimberelimab for use in prevention or treatment of head and neck cancer, which is used so as to be administered to a patient with head and neck cancer in combination with futibatinib or a pharmaceutically acceptable salt thereof, and domvanalimab; and
   (iii) domvanalimab for use in prevention or treatment of head and neck cancer, which is used so as to be administered to a patient with head and neck cancer in combination with futibatinib or a pharmaceutically acceptable salt thereof, and zimberelimab.
Item 40-5. Use of any one of the following (i) to (iii) (provided that use of pembrolizumab and use of a compound used so as to be administered in combination with pembrolizumab are excluded) :
   (i) use of futibatinib or a pharmaceutically acceptable salt thereof for preventing or treating head and neck cancer, which is used so as to be administered to a patient with head and neck cancer in combination with zimberelimab and domvanalimab;
   (ii) use of zimberelimab for preventing or treating head and neck cancer, which is used so as to be administered to a patient with head and neck cancer in combination with futibatinib or a pharmaceutically acceptable salt thereof, and domvanalimab; and
   (iii) use of domvanalimab for preventing or treating head and neck cancer, which is used so as to be administered to a patient with head and neck cancer in combination with futibatinib or a pharmaceutically acceptable salt thereof, and zimberelimab.
Item 40-6. A commercial package of any one of the following (i) to (iii) (provided that a commercial package used so as to be administered in combination with a pharmaceutical composition comprising pembrolizumab as an active ingredient and pembrolizumab is excluded):
   (i) the commercial package comprising futibatinib or a pharmaceutically acceptable salt thereof as an active ingredient, together with an instruction manual for use thereof for preventing or treating head and neck cancer in a subject, which is used so as to be administered to a patient with head and neck cancer in combination with zimberelimab and domvanalimab;
   (ii) the commercial package comprising zimberelimab as an active ingredient, together with an instruction manual for use thereof for preventing or treating head and neck cancer in a subject, which is used so as to be administered to a patient with head and neck cancer in combination with futibatinib or a pharmaceutically acceptable salt thereof, and domvanalimab; and
   (iii) the commercial package comprising domvanalimab as an active ingredient, together with an instruction manual for use thereof for preventing or treating head and neck cancer in a subject, which is used so as to be administered to a patient with head and neck cancer in combination with futibatinib or a pharmaceutically acceptable salt thereof, and zimberelimab.
Item 40-7. A method according to any one of the following (i) to (iii) (provided that a method comprising administering pembrolizumab and a method comprising administering an antitumor agent to a subject who has been administered, is administered simultaneously, or is to be administered pembrolizumab are excluded):
   (i) a method of preventing or treating head and neck cancer, comprising administering an effective amount of futibatinib or a pharmaceutically acceptable salt thereof to a subject in need thereof, in which the subject has been administered, is administered simultaneously, or is to be administered zimberelimab, and the subject has been administered, is administered simultaneously, or is to be administered domvanalimab;
   (ii) a method of preventing or treating head and neck cancer, comprising administering an effective amount of zimberelimab to a subject in need thereof, in which the subject has been administered, is administered simultaneously, or is to be administered futibatinib or a pharmaceutically acceptable salt thereof, and the subject has been administered, is administered simultaneously, or is to be administered domvanalimab; and
   (iii) a method of preventing or treating head and neck cancer, comprising administering an effective amount of domvanalimab to a subject in need thereof, in which the subject has been administered, is administered simultaneously, or is to be administered futibatinib or a pharmaceutically acceptable salt thereof, and the subject has been administered, is administered simultaneously, or is to be administered zimberelimab.

Item 41. The antitumor agent, the pharmaceutical composition, the use, the compound for use or a salt thereof, the commercial package, or the method according to Item 40, wherein a 21-day administration cycle in which 12 mg/dose, 16 mg/dose, or 20 mg/dose of futibatinib is administered daily once a day, 360 mg/dose of zimberelimab is administered once, and 1200 mg/dose of domvanalimab is administered once is repeated once or twice or more.

Item 42. The antitumor agent, the pharmaceutical composition, the use, the compound for use or a salt thereof, the commercial package, or the method according to Item 40, wherein a 21-day administration cycle in which 12 mg/dose, 16 mg/dose, or 20 mg/dose of futibatinib is administered daily once a day, 360 mg/dose of zimberelimab is administered once on Day 1, and 1200 mg/dose of domvanalimab is administered once on Day 1 is repeated once or twice or more.

Item 43. The antitumor agent, the pharmaceutical composition, the use, the compound for use or a salt thereof, the commercial package, or the method according to any one of Items 40 to 42, wherein the cancer patient is a patient with head and neck cancer who has not been previously treated for advanced cancer.

Item 44. The antitumor agent, the pharmaceutical composition, the use, the compound for use or a salt thereof, the commercial package, or the method according to any one of Items 1 to 27, which is the following antitumor agent according to Item 44-1, pharmaceutical composition according to Item 44-2, use according to Item 44-3, compound for use or a salt thereof according to Item 44-4, use according to Item 44-5, commercial package according to Item 44-6, or method according to Item 44-7:
Item 44-1. An antitumor agent of any one of the following (i) to (iv) (provided that an antitumor agent comprising pembrolizumab as an active ingredient and an antitumor agent used so as to be administered in combination with pembrolizumab are excluded):
   (i) the antitumor agent comprising, as an active ingredient, futibatinib or a pharmaceutically acceptable salt thereof, which is used so as to be administered to a patient with non-small cell lung cancer in combination with zimberelimab, albumin-bound paclitaxel, and carboplatin;
   (ii) the antitumor agent comprising, as an active ingredient, zimberelimab, which is used so as to be administered to a patient with non-small cell lung cancer in combination with futibatinib or a pharmaceutically acceptable salt thereof, albumin-bound paclitaxel, and carboplatin;
   (iii) the antitumor agent comprising, as an active ingredient, albumin-bound paclitaxel, which is used so as to be administered to a patient with non-small cell lung cancer in combination with futibatinib or a pharmaceutically acceptable salt thereof, zimberelimab, and carboplatin; and
   (iv) the antitumor agent comprising, as an active ingredient, carboplatin, which is used so as to be administered to a patient with non-small cell lung cancer in combination with futibatinib or a pharmaceutically acceptable salt thereof, zimberelimab, and albumin-bound paclitaxel.
Item 44-2. A pharmaceutical composition for preventing or treating non-small cell lung cancer of any one of the following (i) to (iv) (provided that a pharmaceutical composition comprising pembrolizumab as an active ingredient and a pharmaceutical composition used so as to be administered in combination with pembrolizumab are excluded):
   (i) the pharmaceutical composition comprising, as an active ingredient, futibatinib or a pharmaceutically acceptable salt thereof and further comprising a pharmaceutical carrier, which is used so as to be administered to a patient with non-small cell lung cancer in combination with zimberelimab, albumin-bound paclitaxel, and carboplatin;
   (ii) the pharmaceutical composition comprising, as an active ingredient, zimberelimab and further comprising a pharmaceutical carrier, which is used so as to be administered to a patient with non-small cell lung cancer in combination with futibatinib or a pharmaceutically acceptable salt thereof, albumin-bound paclitaxel, and carboplatin;
   (iii) the pharmaceutical composition comprising, as an active ingredient, albumin-bound paclitaxel and further comprising a pharmaceutical carrier, which is used so as to be administered to a patient with non-small cell lung cancer in combination with futibatinib or a pharmaceutically acceptable salt thereof, zimberelimab, and carboplatin; and
   (iv) the pharmaceutical composition comprising, as an active ingredient, carboplatin and further comprising a pharmaceutical carrier, which is used so as to be administered to a patient with non-small cell lung cancer in combination with futibatinib or a pharmaceutically acceptable salt thereof, zimberelimab, and albumin-bound paclitaxel.
Item 44-3. Use of any one of the following (i) to (iv) (provided that use for producing an antitumor agent comprising pembrolizumab as an active ingredient and use for producing an antitumor agent used so as to be administered in combination with pembrolizumab are excluded):
   (i) use of futibatinib or a pharmaceutically acceptable salt thereof, for producing an antitumor agent used so as to be administered to a patient with non-small cell lung cancer in combination with zimberelimab, albumin-bound paclitaxel, and carboplatin;
   (ii) use of zimberelimab, for producing an antitumor agent used so as to be administered to a patient with non-small cell lung cancer in combination with futibatinib or a pharmaceutically acceptable salt thereof, albumin-bound paclitaxel, and carboplatin;
   (iii) use of albumin-bound paclitaxel, for producing an antitumor agent used so as to be administered to a patient with non-small cell lung cancer in combination with futibatinib or a pharmaceutically acceptable salt thereof, zimberelimab, and carboplatin; and
   (iv) use of carboplatin, for producing an antitumor agent used so as to be administered to a patient with non-small cell lung cancer in combination with futibatinib or a pharmaceutically acceptable salt thereof, zimberelimab, and albumin-bound paclitaxel.
Item 44-4. A compound for use or a salt thereof according to any one of the following (i) to (iv) (provided that a compound used so as to be administered in combination with pembrolizumab and pembrolizumab for use are excluded):
   (i) futibatinib or a pharmaceutically acceptable salt thereof for use in prevention or treatment of non-small cell lung cancer, which is used so as to be administered to a patient with non-small cell lung cancer in combination with zimberelimab, albumin-bound paclitaxel, and carboplatin;
   (ii) zimberelimab for use in prevention or treatment of non-small cell lung cancer, which is used so as to be administered to a patient with non-small cell lung cancer in combination with futibatinib or a pharmaceutically acceptable salt thereof, albumin-bound paclitaxel, and carboplatin;
   (iii) albumin-bound paclitaxel for use in prevention or treatment of non-small cell lung cancer, which is used so as to be administered to a patient with non-small cell lung cancer in combination with futibatinib or a pharmaceutically acceptable salt thereof, zimberelimab, and carboplatin; and
   (iv) carboplatin for use in prevention or treatment of non-small cell lung cancer, which is used so as to be administered to a patient with non-small cell lung cancer in combination with futibatinib or a pharmaceutically acceptable salt thereof, zimberelimab, and albumin-bound paclitaxel.
Item 44-5. Use of any one of the following (i) to (iv) (provided that use of pembrolizumab and use of a compound used so as to be administered in combination with pembrolizumab are excluded):
   (i) use of futibatinib or a pharmaceutically acceptable salt thereof for preventing or treating non-small cell lung cancer, which is used so as to be administered to a patient with non-small cell lung cancer in combination with zimberelimab, albumin-bound paclitaxel, and carboplatin;
   (ii) use of zimberelimab for preventing or treating non-small cell lung cancer, which is used so as to be administered to a patient with non-small cell lung cancer in combination with futibatinib or a pharmaceutically acceptable salt thereof, albumin-bound paclitaxel, and carboplatin;
   (iii) use of albumin-bound paclitaxel for preventing or treating non-small cell lung cancer, which is used so as to be administered to a patient with non-small cell lung cancer in combination with futibatinib or a pharmaceutically acceptable salt thereof, zimberelimab, and carboplatin; and
   (iv) use of carboplatin for preventing or treating non-small cell lung cancer, which is used so as to be administered to a patient with non-small cell lung cancer in combination with futibatinib or a pharmaceutically acceptable salt thereof, zimberelimab, and albumin-bound paclitaxel.
Item 44-6. A commercial package of any one of the following (i) to (iv) (provided that a commercial package used so as to be administered in combination with a pharmaceutical composition comprising pembrolizumab as an active ingredient and pembrolizumab is excluded):
   (i) the commercial package comprising futibatinib or a pharmaceutically acceptable salt thereof as an active ingredient, together with an instruction manual for use thereof for preventing or treating non-small cell lung cancer in a subject, which is used so as to be administered to a patient with non-small cell lung cancer in combination with zimberelimab, albumin-bound paclitaxel, and carboplatin;
   (ii) the commercial package comprising zimberelimab as an active ingredient, together with an instruction manual for use thereof for preventing or treating non-small cell lung cancer in a subject, which is used so as to be administered to a patient with non-small cell lung cancer in combination with futibatinib or a pharmaceutically acceptable salt thereof, albumin-bound paclitaxel, and carboplatin;
   (iii) the commercial package comprising albumin-bound paclitaxel as an active ingredient, together with an instruction manual for use thereof for preventing or treating non-small cell lung cancer in a subject, which is used so as to be administered to a patient with non-small cell lung cancer in combination with futibatinib or a pharmaceutically acceptable salt thereof, zimberelimab, and carboplatin; and
   (iv) the commercial package comprising carboplatin as an active ingredient, together with an instruction manual for use thereof for preventing or treating non-small cell lung cancer in a subject, which is used so as to be administered to a patient with non-small cell lung cancer in combination with futibatinib or a pharmaceutically acceptable salt thereof, zimberelimab, and albumin-bound paclitaxel.
Item 44-7. A method according to any one of the following (i) to (iv) (provided that a method comprising administering pembrolizumab and a method comprising administering an antitumor agent to a subject who has been administered, is administered simultaneously, or is to be administered pembrolizumab are excluded):
   (i) a method of preventing or treating non-small cell lung cancer, comprising administering an effective amount of futibatinib or a pharmaceutically acceptable salt thereof to a subject in need thereof, in which the subject has been administered, is administered simultaneously, or is to be administered zimberelimab, has been administered, is administered simultaneously, or is to be administered albumin-bound paclitaxel, or has been administered, is administered simultaneously, or is to be administered carboplatin;
   (ii) a method of preventing or treating non-small cell lung cancer, comprising administering an effective amount of zimberelimab to a subject in need thereof, in which the subject has been administered, is administered simultaneously, or is to be administered futibatinib or a pharmaceutically acceptable salt thereof, has been administered, is administered simultaneously, or is to be administered albumin-bound paclitaxel, or has been administered, is administered simultaneously, or is to be administered carboplatin;
   (iii) a method of preventing or treating non-small cell lung cancer, comprising administering an effective amount of albumin-bound paclitaxel to a subject in need thereof, in which the subject has been administered, is administered simultaneously, or is to be administered futibatinib or a pharmaceutically acceptable salt thereof, has been administered, is administered simultaneously, or is to be administered zimberelimab, or has been administered, is administered simultaneously, or is to be administered carboplatin; and
   (iv) a method of preventing or treating non-small cell lung cancer, comprising administering an effective amount of carboplatin to a subject in need thereof, in which the subject has been administered, is administered simultaneously, or is to be administered futibatinib or a pharmaceutically acceptable salt thereof, has been administered, is administered simultaneously, or is to be administered zimberelimab, or has been administered, is administered simultaneously, or is to be administered albumin-bound paclitaxel.

Item 45. The antitumor agent, the pharmaceutical composition, the use, the compound for use or a salt thereof, the commercial package, or the method according to Item 44, wherein a 21-day administration cycle in which 12 mg/dose, 16 mg/dose, or 20 mg/dose of futibatinib is administered daily once a day, 360 mg/dose of zimberelimab is administered once, 100 mg/m²/dose of albumin-bound paclitaxel is administered three times, and AUC 6 carboplatin is administered once is repeated once or twice or more.

Item 46. The antitumor agent, the pharmaceutical composition, the use, the compound for use or a salt thereof, the commercial package, or the method according to Item 44, wherein a 21-day administration cycle in which 12 mg/dose, 16 mg/dose, or 20 mg/dose of futibatinib is administered daily once a day, 360 mg/dose of zimberelimab is administered on Day 1, 100 mg/m²/dose of albumin-bound paclitaxel is administered once on Day 1, Day 8, and Day 15, and AUC 6 carboplatin is administered on Day 1 is repeated once or twice or more.

Item 47. The antitumor agent, the pharmaceutical composition, the use, the compound for use or a salt thereof, the commercial package, or the method according to any one of Items 44 to 46, wherein the cancer patient is a patient with non-small cell lung cancer who has not been previously treated for advanced cancer.

Item 48. The antitumor agent, the pharmaceutical composition, the use, the compound for use or a salt thereof, the commercial package, or the method according to any one of Items 1 to 27, which is the following antitumor agent according to Item 48-1, pharmaceutical composition according to Item 48-2, use according to Item 48-3, compound for use or a salt thereof according to Item 48-4, use according to Item 48-5, commercial package according to Item 48-6, or method according to Item 48-7:
Item 48-1. An antitumor agent of any one of the following (i) to (iv) (provided that an antitumor agent comprising pembrolizumab as an active ingredient and an antitumor agent used so as to be administered in combination with pembrolizumab are excluded):
   (i) the antitumor agent comprising, as an active ingredient, futibatinib or a pharmaceutically acceptable salt thereof, which is used so as to be administered to a patient with biliary tract cancer in combination with zimberelimab, cisplatin, and gemcitabine;
   (ii) the antitumor agent comprising, as an active ingredient, zimberelimab, which is used so as to be administered to a patient with biliary tract cancer in combination with futibatinib or a pharmaceutically acceptable salt thereof, cisplatin, and gemcitabine;
   (iii) the antitumor agent comprising, as an active ingredient, gemcitabine, which is used so as to be administered to a patient with biliary tract cancer in combination with futibatinib or a pharmaceutically acceptable salt thereof, zimberelimab, and cisplatin; and
   (iv) the antitumor agent comprising, as an active ingredient, cisplatin, which is used so as to be administered to a patient with biliary tract cancer in combination with futibatinib or a pharmaceutically acceptable salt thereof, zimberelimab, and gemcitabine.
Item 48-2. A pharmaceutical composition for preventing or treating biliary tract cancer of any one of the following (i) to (iv) (provided that a pharmaceutical composition comprising pembrolizumab as an active ingredient and a pharmaceutical composition used so as to be administered in combination with pembrolizumab are excluded):
   (i) the pharmaceutical composition comprising, as an active ingredient, futibatinib or a pharmaceutically acceptable salt thereof and further comprising a pharmaceutical carrier, which is used so as to be administered to a patient with biliary tract cancer in combination with zimberelimab, cisplatin, and gemcitabine;
   (ii) the pharmaceutical composition comprising, as an active ingredient, zimberelimab and further comprising a pharmaceutical carrier, which is used so as to be administered to a patient with biliary tract cancer in combination with futibatinib or a pharmaceutically acceptable salt thereof, cisplatin, and gemcitabine;
   (iii) the pharmaceutical composition comprising, as an active ingredient, gemcitabine and further comprising a pharmaceutical carrier, which is used so as to be administered to a patient with biliary tract cancer in combination with futibatinib or a pharmaceutically acceptable salt thereof, zimberelimab, and cisplatin; and
   (iv) the pharmaceutical composition comprising, as an active ingredient, cisplatin and further comprising a pharmaceutical carrier, which is used so as to be administered to a patient with biliary tract cancer in combination with futibatinib or a pharmaceutically acceptable salt thereof, zimberelimab, and gemcitabine.
Item 48-3. Use of any one of the following (i) to (iv) (provided that use for producing an antitumor agent comprising pembrolizumab as an active ingredient and use for producing an antitumor agent used so as to be administered in combination with pembrolizumab are excluded):
   (i) use of futibatinib or a pharmaceutically acceptable salt thereof, for producing an antitumor agent used so as to be administered to a patient with biliary tract cancer in combination with zimberelimab, cisplatin, and gemcitabine;
   (ii) use of zimberelimab, for producing an antitumor agent used so as to be administered to a patient with biliary tract cancer in combination with futibatinib or a pharmaceutically acceptable salt thereof, cisplatin, and gemcitabine;
   (iii) use of gemcitabine, for producing an antitumor agent used so as to be administered to a patient with biliary tract cancer in combination with futibatinib or a pharmaceutically acceptable salt thereof, zimberelimab, and cisplatin; and
   (iv) use of cisplatin, for producing an antitumor agent used so as to be administered to a patient with biliary tract cancer in combination with futibatinib or a pharmaceutically acceptable salt thereof, zimberelimab, and gemcitabine.
Item 48-4. A compound for use or a salt thereof according to any one of the following (i) to (iv) (provided that a compound used so as to be administered in combination with pembrolizumab and pembrolizumab for use are excluded):
   (i) futibatinib or a pharmaceutically acceptable salt thereof for use in prevention or treatment of biliary tract cancer, which is used so as to be administered to a patient with biliary tract cancer in combination with zimberelimab, cisplatin, and gemcitabine;
   (ii) zimberelimab for use in prevention or treatment of biliary tract cancer, which is used so as to be administered to a patient with biliary tract cancer in combination with futibatinib or a pharmaceutically acceptable salt thereof, cisplatin, and gemcitabine;
   (iii) gemcitabine for use in prevention or treatment of biliary tract cancer, which is used so as to be administered to a patient with biliary tract cancer in combination with futibatinib or a pharmaceutically acceptable salt thereof, zimberelimab, and cisplatin; and
   (iv) cisplatin for use in prevention or treatment of biliary tract cancer, which is used so as to be administered to a patient with biliary tract cancer in combination with futibatinib or a pharmaceutically acceptable salt thereof, zimberelimab, and gemcitabine.
Item 48-5. Use of any one of the following (i) to (iv) (provided that use of pembrolizumab and use of a compound used so as to be administered in combination with pembrolizumab are excluded) :
   (i) use of futibatinib or a pharmaceutically acceptable salt thereof for preventing or treating biliary tract cancer, which is used so as to be administered to a patient with biliary tract cancer in combination with zimberelimab, cisplatin, and gemcitabine;
   (ii) use of zimberelimab for preventing or treating biliary tract cancer, which is used so as to be administered to a patient with biliary tract cancer in combination with futibatinib or a pharmaceutically acceptable salt thereof, cisplatin, and gemcitabine;
   (iii) use of gemcitabine for preventing or treating biliary tract cancer, which is used so as to be administered to a patient with biliary tract cancer in combination with futibatinib or a pharmaceutically acceptable salt thereof, zimberelimab, and cisplatin; and
   (iv) use of cisplatin for preventing or treating biliary tract cancer, which is used so as to be administered to a patient with biliary tract cancer in combination with futibatinib or a pharmaceutically acceptable salt thereof, zimberelimab, and gemcitabine.
Item 48-6. A commercial package of any one of the following (i) to (iv) (provided that a commercial package used so as to be administered in combination with a pharmaceutical composition comprising pembrolizumab as an active ingredient and pembrolizumab is excluded):
   (i) the commercial package comprising futibatinib or a pharmaceutically acceptable salt thereof as an active ingredient, together with an instruction manual for use thereof for preventing or treating biliary tract cancer in a subject, which is used so as to be administered to a patient with biliary tract cancer in combination with zimberelimab, cisplatin, and gemcitabine;
   (ii) the commercial package comprising zimberelimab as an active ingredient, together with an instruction manual for use thereof for preventing or treating biliary tract cancer in a subject, which is used so as to be administered to a patient with biliary tract cancer in combination with futibatinib or a pharmaceutically acceptable salt thereof, cisplatin, and gemcitabine;
   (iii) the commercial package comprising gemcitabine as an active ingredient, together with an instruction manual for use thereof for preventing or treating biliary tract cancer in a subject, which is used so as to be administered to a patient with biliary tract cancer in combination with futibatinib or a pharmaceutically acceptable salt thereof, zimberelimab, and cisplatin; and
   (iv) the commercial package comprising cisplatin as an active ingredient, together with an instruction manual for use thereof for preventing or treating biliary tract cancer in a subject, which is used so as to be administered to a patient with biliary tract cancer in combination with futibatinib or a pharmaceutically acceptable salt thereof, zimberelimab, and gemcitabine.
Item 48-7. A method according to any one of the following (i) to (iv) (provided that a method comprising administering pembrolizumab and a method comprising administering an antitumor agent to a subject who has been administered, is administered simultaneously, or is to be administered pembrolizumab are excluded):
   (i) a method of preventing or treating biliary tract cancer, comprising administering an effective amount of futibatinib or a pharmaceutically acceptable salt thereof to a subject in need thereof, in which the subject has been administered, is administered simultaneously, or is to be administered zimberelimab, has been administered, is administered simultaneously, or is to be administered cisplatin, or has been administered, is administered simultaneously, or is to be administered gemcitabine;
   (ii) a method of preventing or treating biliary tract cancer, comprising administering an effective amount of zimberelimab to a subject in need thereof, in which the subject has been administered, is administered simultaneously, or is to be administered futibatinib or a pharmaceutically acceptable salt thereof, has been administered, is administered simultaneously, or is to be administered cisplatin, or has been administered, is administered simultaneously, or is to be administered gemcitabine;
   (iii) a method of preventing or treating biliary tract cancer, comprising administering an effective amount of gemcitabine to a subject in need thereof, in which the subject has been administered, is administered simultaneously, or is to be administered futibatinib or a pharmaceutically acceptable salt thereof, has been administered, is administered simultaneously, or is to be administered zimberelimab, or has been administered, is administered simultaneously, or is to be administered cisplatin; and
   (iv) a method of preventing or treating biliary tract cancer, comprising administering an effective amount of cisplatin to a subject in need thereof, in which the subject has been administered, is administered simultaneously, or is to be administered futibatinib or a pharmaceutically acceptable salt thereof, has been administered, is administered simultaneously, or is to be administered zimberelimab, or has been administered, is administered simultaneously, or is to be administered gemcitabine.

Item 49. The antitumor agent, the pharmaceutical composition, the use, the compound for use or a salt thereof, the commercial package, or the method according to Item 48, wherein a 21-day administration cycle in which 12 mg/dose, 16 mg/dose, or 20 mg/dose of futibatinib is administered daily once a day, 360 mg/dose of zimberelimab is administered once, 25 mg/m²/dose of cisplatin is administered twice, and 1000 mg/m²/day of gemcitabine is administered twice is repeated once or twice or more.

Item 50. The antitumor agent, the pharmaceutical composition, the use, the compound for use or a salt thereof, the commercial package, or the method according to Item 48, wherein a 21-day administration cycle in which 12 mg/dose, 16 mg/dose, or 20 mg/dose of futibatinib is administered daily once a day, 360 mg/dose of zimberelimab is administered on Day 1, 25 mg/m²/dose of cisplatin is administered once on Day 1 and Day 8, and 1000 mg/m²/day of gemcitabine is administered once on Day 1 and Day 8 is repeated once or twice or more.

Item 51. The antitumor agent, the pharmaceutical composition, the use, the compound for use or a salt thereof, the commercial package, or the method according to any one of Items 48 to 50, wherein the cancer patient is a patient with biliary tract cancer who has not been previously treated for advanced cancer.

Item 52. The antitumor agent, the pharmaceutical composition, the use, the compound for use or a salt thereof, the commercial package, or the method according to any one of Items 1 to 27, which is the following antitumor agent according to Item 52-1, pharmaceutical composition according to Item 52-2, use according to Item 52-3, compound for use or a salt thereof according to Item 52-4, use according to Item 52-5, commercial package according to Item 52-6, or method according to Item 52-7:
Item 52-1. An antitumor agent of any one of the following (i) to (iv) (provided that an antitumor agent comprising pembrolizumab as an active ingredient and an antitumor agent used so as to be administered in combination with pembrolizumab are excluded):
   (i) the antitumor agent comprising, as an active ingredient, futibatinib or a pharmaceutically acceptable salt thereof, which is used so as to be administered to a patient with pancreatic cancer in combination with zimberelimab, albumin-bound paclitaxel, and gemcitabine;
   (ii) the antitumor agent comprising, as an active ingredient, zimberelimab, which is used so as to be administered to a patient with pancreatic cancer in combination with futibatinib or a pharmaceutically acceptable salt thereof, albumin-bound paclitaxel, and gemcitabine;
   (iii) the antitumor agent comprising, as an active ingredient, gemcitabine, which is used so as to be administered to a patient with pancreatic cancer in combination with futibatinib or a pharmaceutically acceptable salt thereof, zimberelimab, and albumin-bound paclitaxel; and
   (iv) the antitumor agent comprising, as an active ingredient, albumin-bound paclitaxel, which is used so as to be administered to a patient with pancreatic cancer in combination with futibatinib or a pharmaceutically acceptable salt thereof, zimberelimab, and gemcitabine.
Item 52-2. A pharmaceutical composition for preventing or treating pancreatic cancer of any one of the following (i) to (iv) (provided that a pharmaceutical composition comprising pembrolizumab as an active ingredient and a pharmaceutical composition used so as to be administered in combination with pembrolizumab are excluded):
   (i) the pharmaceutical composition comprising, as an active ingredient, futibatinib or a pharmaceutically acceptable salt thereof and further comprising a pharmaceutical carrier, which is used so as to be administered to a patient with pancreatic cancer in combination with zimberelimab, albumin-bound paclitaxel, and gemcitabine;
   (ii) the pharmaceutical composition comprising, as an active ingredient, zimberelimab and further comprising a pharmaceutical carrier, which is used so as to be administered to a patient with pancreatic cancer in combination with futibatinib or a pharmaceutically acceptable salt thereof, albumin-bound paclitaxel, and gemcitabine;
   (iii) the pharmaceutical composition comprising, as an active ingredient, gemcitabine and further comprising a pharmaceutical carrier, which is used so as to be administered to a patient with pancreatic cancer in combination with futibatinib or a pharmaceutically acceptable salt thereof, zimberelimab, and albumin-bound paclitaxel; and
   (iv) the pharmaceutical composition comprising, as an active ingredient, albumin-bound paclitaxel and further comprising a pharmaceutical carrier, which is used so as to be administered to a patient with pancreatic cancer in combination with futibatinib or a pharmaceutically acceptable salt thereof, zimberelimab, and gemcitabine.
Item 52-3. Use of any one of the following (i) to (iv) (provided that use for producing an antitumor agent comprising pembrolizumab as an active ingredient and use for producing an antitumor agent used so as to be administered in combination with pembrolizumab are excluded):
   (i) use of futibatinib or a pharmaceutically acceptable salt thereof, for producing an antitumor agent used so as to be administered to a patient with pancreatic cancer in combination with zimberelimab, albumin-bound paclitaxel, and gemcitabine;
   (ii) use of zimberelimab, for producing an antitumor agent used so as to be administered to a patient with pancreatic cancer in combination with futibatinib or a pharmaceutically acceptable salt thereof, albumin-bound paclitaxel, and gemcitabine;
   (iii) use of gemcitabine, for producing an antitumor agent used so as to be administered to a patient with pancreatic cancer in combination with futibatinib or a pharmaceutically acceptable salt thereof, zimberelimab, and albumin-bound paclitaxel; and
   (iv) use of albumin-bound paclitaxel, for producing an antitumor agent used so as to be administered to a patient with pancreatic cancer in combination with futibatinib or a pharmaceutically acceptable salt thereof, zimberelimab, and gemcitabine.
Item 52-4. A compound for use or a salt thereof according to any one of the following (i) to (iv) (provided that a compound used so as to be administered in combination with pembrolizumab and pembrolizumab for use are excluded):
   (i) futibatinib or a pharmaceutically acceptable salt thereof for use in prevention or treatment of pancreatic cancer, which is used so as to be administered to a patient with pancreatic cancer in combination with zimberelimab, albumin-bound paclitaxel, and gemcitabine;
   (ii) zimberelimab for use in prevention or treatment of pancreatic cancer, which is used so as to be administered to a patient with pancreatic cancer in combination with futibatinib or a pharmaceutically acceptable salt thereof, albumin-bound paclitaxel, and gemcitabine;
   (iii) gemcitabine for use in prevention or treatment of pancreatic cancer, which is used so as to be administered to a patient with pancreatic cancer in combination with futibatinib or a pharmaceutically acceptable salt thereof, zimberelimab, and albumin-bound paclitaxel; and
   (iv) albumin-bound paclitaxel for use in prevention or treatment of pancreatic cancer, which is used so as to be administered to a patient with pancreatic cancer in combination with futibatinib or a pharmaceutically acceptable salt thereof, zimberelimab, and gemcitabine.
Item 52-5. Use of any one of the following (i) to (iv) (provided that use of pembrolizumab and use of a compound used so as to be administered in combination with pembrolizumab are excluded) :
   (i) use of futibatinib or a pharmaceutically acceptable salt thereof for preventing or treating pancreatic cancer, which is used so as to be administered to a patient with pancreatic cancer in combination with zimberelimab, albumin-bound paclitaxel, and gemcitabine;
   (ii) use of zimberelimab for preventing or treating pancreatic cancer, which is used so as to be administered to a patient with pancreatic cancer in combination with futibatinib or a pharmaceutically acceptable salt thereof, albumin-bound paclitaxel, and gemcitabine;
   (iii) use of gemcitabine for preventing or treating pancreatic cancer, which is used so as to be administered to a patient with pancreatic cancer in combination with futibatinib or a pharmaceutically acceptable salt thereof, zimberelimab, and albumin-bound paclitaxel; and
   (iv) use of albumin-bound paclitaxel for preventing or treating pancreatic cancer, which is used so as to be administered to a patient with pancreatic cancer in combination with futibatinib or a pharmaceutically acceptable salt thereof, zimberelimab, and gemcitabine.
Item 52-6. A commercial package of any one of the following (i) to (iv) (provided that a commercial package used so as to be administered in combination with a pharmaceutical composition comprising pembrolizumab as an active ingredient and pembrolizumab is excluded) :
   (i) the commercial package comprising futibatinib or a pharmaceutically acceptable salt thereof as an active ingredient, together with an instruction manual for use thereof for preventing or treating pancreatic cancer in a subject, which is used so as to be administered to a patient with pancreatic cancer in combination with zimberelimab, albumin-bound paclitaxel, and gemcitabine;
   (ii) the commercial package comprising zimberelimab or a pharmaceutically acceptable salt thereof as an active ingredient, together with an instruction manual for use thereof for preventing or treating pancreatic cancer in a subject, which is used so as to be administered to a patient with pancreatic cancer in combination with futibatinib or a pharmaceutically acceptable salt thereof, albumin-bound paclitaxel, and gemcitabine;
   (iii) the commercial package comprising gemcitabine as an active ingredient, together with an instruction manual for use thereof for preventing or treating pancreatic cancer in a subject, which is used so as to be administered to a patient with pancreatic cancer in combination with futibatinib or a pharmaceutically acceptable salt thereof, zimberelimab, and albumin-bound paclitaxel; and
   (iv) the commercial package comprising albumin-bound paclitaxel as an active ingredient, together with an instruction manual for use thereof for preventing or treating pancreatic cancer in a subject, which is used so as to be administered to a patient with pancreatic cancer in combination with futibatinib or a pharmaceutically acceptable salt thereof, zimberelimab, and gemcitabine.
Item 52-7. A method according to any one of the following (i) to (iv) (provided that a method comprising administering pembrolizumab and a method comprising administering an antitumor agent to a subject who has been administered, is administered simultaneously, or is to be administered pembrolizumab are excluded):
   (i) a method of preventing or treating pancreatic cancer, comprising administering an effective amount of futibatinib or a pharmaceutically acceptable salt thereof to a subject in need thereof, in which the subject has been administered, is administered simultaneously, or is to be administered zimberelimab, has been administered, is administered simultaneously, or is to be administered albumin-bound paclitaxel, or has been administered, is administered simultaneously, or is to be administered gemcitabine;
   (ii) a method of preventing or treating pancreatic cancer, comprising administering an effective amount of zimberelimab to a subject in need thereof, in which the subject has been administered, is administered simultaneously, or is to be administered futibatinib or a pharmaceutically acceptable salt thereof, has been administered, is administered simultaneously, or is to be administered albumin-bound paclitaxel, or has been administered, is administered simultaneously, or is to be administered gemcitabine;
   (iii) a method of preventing or treating pancreatic cancer, comprising administering an effective amount of gemcitabine to a subject in need thereof, in which the subject has been administered, is administered simultaneously, or is to be administered futibatinib or a pharmaceutically acceptable salt thereof, has been administered, is administered simultaneously, or is to be administered zimberelimab, or has been administered, is administered simultaneously, or is to be administered albumin-bound paclitaxel; and
   (iv) a method of preventing or treating pancreatic cancer, comprising administering an effective amount of albumin-bound paclitaxel to a subject in need thereof, in which the subject has been administered, is administered simultaneously, or is to be administered futibatinib or a pharmaceutically acceptable salt thereof, has been administered, is administered simultaneously, or is to be administered zimberelimab, or has been administered, is administered simultaneously, or is to be administered gemcitabine.

Item 53. The antitumor agent, the pharmaceutical composition, the use, the compound for use or a salt thereof, the commercial package, or the method according to Item 52, wherein a 28-day administration cycle in which 12 mg/dose, 16 mg/dose, or 20 mg/dose of futibatinib is administered daily once a day, 240 mg/dose of zimberelimab is administered twice, 125 mg/m²/dose of albumin-bound paclitaxel is administered three times, and 1000 mg/m²/dose of gemcitabine is administered three times is repeated once or twice or more.

Item 54. The antitumor agent, the pharmaceutical composition, the use, the compound for use or a salt thereof, the commercial package, or the method according to Item 52, wherein a 28-day administration cycle in which 12 mg/dose, 16 mg/dose, or 20 mg/dose of futibatinib is administered daily once a day, 240 mg/dose of zimberelimab is administered on Day 1 and Day 15, 125 mg/m²/dose of albumin-bound paclitaxel is administered once on Day 1, Day 8, and Day 15, and 1000 mg/m²/dose of gemcitabine is administered once on Day 1, Day 8, and Day 15 is repeated once or twice or more.

Item 55. The antitumor agent, the pharmaceutical composition, the use, the compound for use or a salt thereof, the commercial package, or the method according to any one of Items 52 to 54, wherein the cancer patient is a patient with pancreatic cancer who has not been previously treated for advanced cancer.

### Advantageous Effects of Invention

According to the present invention, it is possible to provide a novel combination therapy that has excellent antitumor effects (such as a tumor reduction effect and a survival prolongation effect) for cancer patients. In particular, the combination therapy of the present invention is effective for patients with lung cancer, esophageal cancer, biliary tract cancer, or head and neck cancer who have not been previously treated.

### Brief Description of Drawings

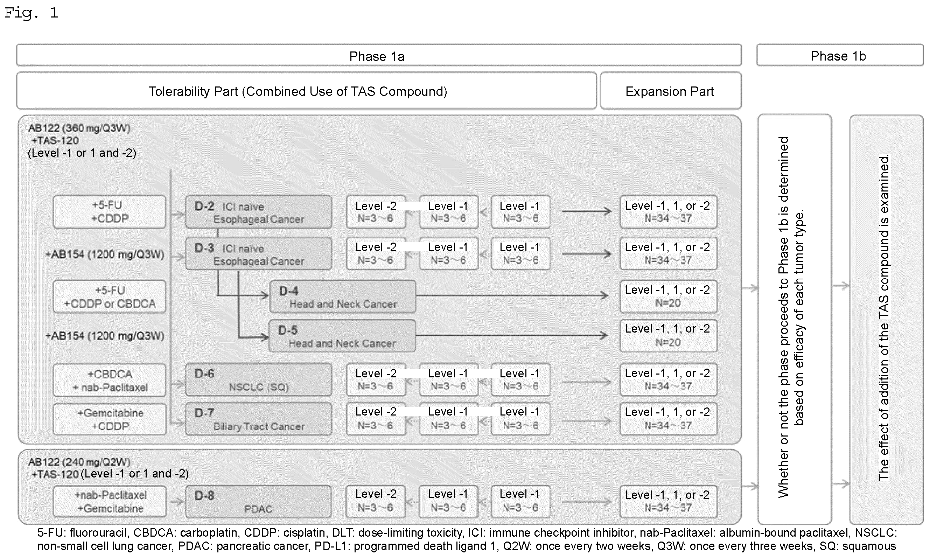

Fig. 1 shows an outline of a clinical trial design of combination therapy comprising futibatinib.

### Description of Embodiments

The present invention relates to the provision of, for example, combined use of futibatinib or a salt thereof, an immune checkpoint inhibitor (except for pembrolizumab and CD155/TIGIT pathway antagonist), and at least one or more other antitumor agents.

In the present invention, futibatinib, which exhibits an excellent antitumor effect when used in combination with an immune checkpoint inhibitor, is a disubstituted benzene alkynyl compound having the following structure. Futibatinib is described as Example Compound 2 in PTL 2 mentioned above. It has been reported that futibatinib or a salt thereof has an excellent FGFR inhibitory effect and inhibits the ability of receptor protein tyrosine kinases of FGFR1, FGFR3, and FGFR4 to phosphorylate tyrosine in the substrate peptide sequence (PTL 2).

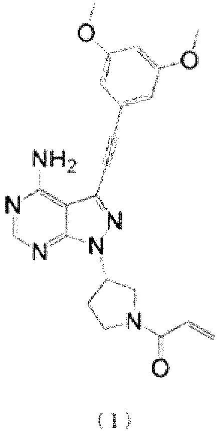

Futibatinib or a pharmaceutically acceptable salt thereof in the present invention described above is not particularly limited, but can be synthesized, for example, based on the production method described in PTL 2.

In the present invention, futibatinib can be used as is or in the form of a pharmaceutically acceptable salt. The pharmaceutically acceptable salt of futibatinib is not particularly limited, and examples thereof include addition salts with inorganic acids such as hydrochloric acid and sulfuric acid, or with organic acids such as acetic acid, citric acid, tartaric acid, and maleic acid, salts with alkali metals such as potassium and sodium, salts with alkaline earth metals such as calcium and magnesium, salts with organic bases such as ammonium salts, ethylamine salts, and arginine salts, and the like.

In the present invention, the immune checkpoint inhibitors act on immune checkpoint molecules, except for pembrolizumab and an anti-TIGIT antibody, and have the effects of inducing antitumor immune responses in the bodies of subjects and preventing tumor immune escape.

Examples of such immune checkpoint inhibitors include substances that promote the function of costimulatory molecules (stimulatory costimulatory molecules), and substances that inhibit the function of coinhibitory molecules (inhibitory costimulatory molecules). Examples of immune checkpoint molecules include B7 family (B7-1, B7-2, PD-L1, PD-L2, etc.), CD28 family (CTLA-4, PD-1, etc.), TNF superfamily (4-1BBL, OX40L), and TNF receptor superfamily (4-1BB, OX40) molecules, and substances that target immune checkpoint molecules can be used as immune checkpoint inhibitors. Examples thereof include PD-1 pathway antagonists, ICOS pathway agonists, CTLA-4 pathway antagonists, CD28 pathway agonists, BTLA pathway antagonists, 4-1BB pathway agonists, CD155/TIGIT pathway antagonists, and the like.

In the present invention, preferred is at least one or more selected from PD-1 pathway antagonists, ICOS pathway agonists, CTLA-4 pathway antagonists, CD28 pathway agonists, and CD155/TIGIT pathway antagonists, more preferred is at least one or more selected from PD-1 pathway antagonists, CTLA-4 pathway antagonists, and CD155/TIGIT pathway antagonists, still more preferred is at least one or more selected from PD-1 pathway antagonists and CD155/TIGIT pathway antagonists, and even more preferred are PD-1 pathway antagonists.

PD-1 pathway antagonists inhibit immunosuppressive signals of PD-1 expressed on T cells and its ligand PD-L1 or PD-L2, and examples thereof include, but are not limited to, anti-PD-1 antibodies, anti-PD-L1 antibodies, anti-PD-L2 antibodies, PD-1 extracellular domains, PD-L1 extracellular domains, PD-L2 extracellular domains, PD-1-Ig (fusion protein of PD-1 extracellular domain and the FC region of Ig), PD-L1-Ig, PD-L2-Ig, PD-1 siRNA, PD-L1 siRNA, PD-L2 siRNA, and the like. Preferred are anti-PD-1 antibodies, anti-PD-L1 antibodies, or anti-PD-L2 antibodies, and more preferred are anti-PD-1 antibodies or anti-PD-L1 antibodies. Particularly preferred among these are anti-PD-1 antibodies.

Further, CTLA-4 pathway antagonists inhibit immunosuppressive signals of CTLA-4 expressed on T cells and its ligands B7-1 (CD80) and B7-2 (CD86), preferred are anti-CTLA-4 antibodies, CTLA-4 extracellular domains, CTLA-4-Ig (fusion protein of a CTLA-4 extracellular domain and the FC region of Ig), anti-B7-1( CD80) antibodies, or anti-B7-2 (CD86) antibodies, and more preferred are anti-CTLA-4 antibodies or CTLA-4-Ig. Particularly preferred among these are anti-CTLA-4 antibodies.

Further, CD155/TIGIT pathway antagonists inhibit immunosuppressive signals of TIGIT expressed on T cells and its ligand CD155, and examples thereof include, but are not limited to, anti-CD155 antibodies and anti-TIGIT antibodies, and the like. Preferred are anti-CD155 antibodies and anti-TIGIT antibodies, and particularly preferred are anti-TIGIT antibodies.

Examples of these antibodies include immunoglobulins (IgA, IgD, IgE, IgG, IgM, IgY, etc.), Fab fragments, F(ab')₂ fragments, single-chain antibody fragments (scFv), single-domain antibodies, and Diabody (Nat. Rev. Immunol., 6: 343-357, 2006), and these include monoclonal antibodies and polyclonal antibodies, such as human antibodies, humanized antibodies, chimeric antibodies, mouse antibodies, llama antibodies, and chicken antibodies. In the present invention, immunoglobulins (preferably IgG etc.) and the like are preferable. Further, in the present invention, human antibodies or humanized antibodies are preferable. In the present invention, monoclonal antibodies are preferable.

Preferred is a humanized IgG monoclonal antibody or a human IgG monoclonal antibody.

In the present invention, preferable anti-PD-1 antibodies include nivolumab, cemiplimab, spartalizumab, tislelizumab, BI754091, dostarlimab, sasanlimab, MGA-012, cetrelimab, AGEN-2034, zimberelimab, camrelizumab, budigalimab, balstilimab, and the like, nivolumab or zimberelimab is preferable, and zimberelimab is particularly preferable.

In the present invention, preferable anti-PD-L1 antibodies include atezolizumab, durvalumab, avelumab, lodapolimab, BGB-A333, and the like.

In the present invention, preferable anti-CTLA-4 antibodies include ipilimumab, tremelimumab, and the like.

In the present invention, preferable CTLA-4-Ig includes abatacept and the like.

In the present invention, preferable anti-TIGIT antibodies include domvanalimab (AB154), AB308, vibostolimab, ociperlimab, tiragolumab, AGEN-1777, HB-0036, HLX-301, ONO-4686, M-6223, PM-1022, Etigilimab, ZG-005, AZD-2936, Belrestotug or JS-006, and the like, domvanalimab, AB308, vibostolimab, ociperlimab, or tiragolumab is preferable, domvanalimab or AB308 is more preferable, and domvanalimab is particularly preferable.

These agonists or antagonists can be produced by a generally known production method.

Further, anti-PD-1 antibodies have already been sold or will be sold as nivolumab or zimberelimab, anti-PD-L1 antibodies have already been sold or will be sold as atezolizumab, durvalumab, or avelumab, anti-CTLA-4 antibodies have already been sold or will be sold as ipilimumab or tremelimumab, CTLA-4-Ig antibodies have already been sold or will be sold as abatacept, and anti-TIGIT antibodies antibodies have already been sold or will be sold as domvanalimab.

In the present invention, the immune checkpoint inhibitors may be used singly or in combination of two or more.

In the present invention, when two or more immune checkpoint inhibitors are used, for example, immune checkpoint inhibitors such as an anti-PD-1 antibody and an anti-CTLA-4 antibody can also be used in combination, or bispecific antibodies that can bind to different immune checkpoint molecules can also be used. Examples of bispecific antibodies include XmAb20717 (PD-1 × CTLA-4), which can bind to both PD-1 and CTLA-4.

In the present invention, the immune checkpoint inhibitor is preferably a PD-1 pathway antagonist, more preferably an anti-PD-1 antibody, further preferably nivolumab or zimberelimab, and particularly preferably zimberelimab.

The present invention exhibits an excellent antitumor effect by using at least three or more agents in combination by adding "other antitumor agent" (additional antitumor agent) to the combination of futibatinib or a pharmaceutically acceptable salt thereof, and at least one or more immune checkpoint inhibitors. Therefore, the other antitumor agent in the present invention is not particularly limited as long as it is futibatinib or a pharmaceutically acceptable salt thereof, or an agent having an antitumor activity (provided that, pembrolizumab is excluded) other than the selected immune checkpoint inhibitor. Note that, the phrase that the other antitumor agent is "other than the selected immune checkpoint inhibitor" means that, for example, when A is selected as the "immune checkpoint inhibitor" in the antitumor agent of the present invention, A is not selected as the "other antitumor agent". In the present invention, the form of the other antitumor agent may be any of a low-molecular-weight compound, an antibody, and a nucleic acid. In the present invention, the other antitumor agents may be used singly or in combination of two or more.

In the present invention, preferable other antitumor agent is a chemotherapeutic agent. Here, in the present invention, the chemotherapeutic agent is preferably at least one selected from the group consisting of an antimetabolite (a purine-based antimetabolite, a pyrimidine-based antimetabolite, a folic acid antimetabolite, etc.), an alkaloid-based antitumor agent, a platinum preparation, an immune checkpoint inhibitor (a PD-1 pathway antagonist, a CTLA-4 pathway antagonist, a CD155/TIGIT pathway antagonist, etc.), a molecular targeting drug (a low-molecular-weight molecular targeting drug/antibody molecular targeting drug, etc.), an antitumor antibiotic, and an alkylating agent, more preferably at least one selected from the group consisting of an antimetabolite (a purine-based antimetabolite, a pyrimidine-based antimetabolite, a folic acid antimetabolite, etc.), an alkaloid-based antitumor agent, a platinum preparation, and an immune checkpoint inhibitor, is further preferably at least one selected from the group consisting of an antimetabolite (a purine-based antimetabolite, a pyrimidine-based antimetabolite, a folic acid antimetabolite, etc.), an alkaloid-based antitumor agent, a platinum preparation, and a CD155/TIGIT pathway antagonist, and is still more preferably at least one selected from the group consisting of an antimetabolite (a purine-based antimetabolite, a pyrimidine-based antimetabolite, a folic acid antimetabolite, etc.), an alkaloid-based antitumor agent, a platinum preparation, and an anti-TIGIT antibody. Further, in one exemplary preferred embodiment of the present invention, at least agents other than the above-described immune checkpoint inhibitor may be used as the chemotherapeutic agent. In such an embodiment, as the chemotherapeutic agent, for example, at least one selected from the group consisting of an antimetabolite (a purine-based antimetabolite, a pyrimidine-based antimetabolite, a folic acid antimetabolite, etc.), an alkaloid-based antitumor agent, a platinum preparation, a molecular targeting drug (a low-molecular-weight molecular targeting drug/antibody molecular targeting drug, etc.), an antitumor antibiotic, and an alkylating agent can be used.

More specifically, preferred is at least one selected from the group consisting of:
a purine-based antimetabolite such as fludarabine, cladribine, or nelarabine;
a pyrimidine-based antimetabolite such as 5-fluorouracil (5-FU), a tegafur/gimeracil/oteracil potassium compound preparation (TS-1 or S-1, trade name: "TS-1"), a tegafur/uracil compound preparation (UFT, trade name: "UFT"), a trifluridine/tipiracil hydrochloride compound preparation (TAS-102, trade name: "LONSURF"), capecitabine, doxifluridine, 5-fluoro-2'-deoxyuridine (FdUrd), gemcitabine, or cytarabine;
a folic acid antimetabolite such as pemetrexed or methotrexate;
an alkaloid-based antitumor agent such as paclitaxel (sold under trade names of "Taxol", "Abraxane", etc., paclitaxel includes derivatives such as albumin-bound paclitaxel (e.g., ABI-007) and PEG-bound paclitaxel), docetaxel (trade name "Taxotere", etc.), cabazitaxel, eribulin, irinotecan, nogitecan, etoposide, vinorelbine, vincristine, or vinblastine;
a platinum preparation such as cisplatin, carboplatin, oxaliplatin, or nedaplatin;
a PD-1 pathway antagonist such as nivolumab, cemiplimab, spartalizumab, tislelizumab, BI754091, dostarlimab, sasanlimab, MGA-012, cetrelimab, AGEN-2034, zimberelimab, camrelizumab, budigalimab, balstilimab, atezolizumab, durvalumab, avelumab, lodapolimab, or BGB-A333;
a CTLA-4 pathway antagonist such as ipilimumab, tremelimumab, or abatacept;
a CD155/TIGIT pathway antagonist such as domvanalimab, AB308, vibostolimab, ociperlimab, tiragolumab, AGEN-1777, HB-0036, HLX-301, ONO-4686, M-6223, PM-1022, Etigilimab, ZG-005, AZD-2936, Belrestotug, or JS-006;
a low-molecular-weight molecular targeting drug such as imatinib, gefitinib, erlotinib, lapatinib, sunitinib, dasatinib, everolimus, temsirolimus, selumetinib, trametinib, sorafenib, afatinib, regorafenib, dabrafenib, vemurafenib, or MK2206;
an antibody molecular targeting drug such as trastuzumab, cetuximab, bevacizumab, panitumumab, veltuzumab, rituximab, or ramucirumab;
an antitumor antibiotic such as doxorubicin, daunorubicin, epirubicin, actinomycin D, or mitomycin C; and
an alkylating agent such as cyclophosphamide, dacarbazine, temozolomide, nimustine, busulfan, procarbazine, or melphalan,
more preferred is at least one selected from the group consisting of fludarabine, cladribine, nelarabine, 5-fluorouracil (5-FU), a tegafur/gimeracil/oteracil potassium compound preparation (TS-1 or S-1, trade name: "TS-1"), a tegafur/uracil compound preparation (UFT, trade name: "UFT"), a trifluridine/tipiracil hydrochloride compound preparation (TAS-102, trade name: "LONSURF"), capecitabine, doxifluridine, 5-fluoro-2'-deoxyuridine (FdUrd), gemcitabine, cytarabine, pemetrexed, methotrexate, paclitaxel, albumin-bound paclitaxel, docetaxel, cabazitaxel, eribulin, irinotecan, nogitecan, etoposide, vinorelbine, vincristine, vinblastine, cisplatin, carboplatin, oxaliplatin, nedaplatin, domvanalimab, AB308, vibostolimab, ociperlimab, and tiragolumab,
further preferred is at least one selected from the group consisting of 5-fluorouracil, gemcitabine, albumin-bound paclitaxel, irinotecan, cisplatin, carboplatin, domvanalimab, and AB308,
still more preferred is at least one selected from the group consisting of 5-fluorouracil, gemcitabine, albumin-bound paclitaxel, cisplatin, carboplatin, and domvanalimab, and
particularly preferred is a combination of 5-fluorouracil and cisplatin, a combination of 5-fluorouracil and carboplatin, a combination of carboplatin and albumin-bound paclitaxel, a combination of cisplatin and gemcitabine, a combination of albumin-bound paclitaxel and gemcitabine, or domvanalimab.

Suitable antitumor agents in the present invention include an antitumor agent of any one of the following (i) to (iii), the antitumor agent not comprising, as an active ingredient, pembrolizumab:
(i) the antitumor agent comprising, as an active ingredient, futibatinib or a pharmaceutically acceptable salt thereof, which is used so as to be administered to a cancer patient in combination with an anti-PD-1 antibody and at least one or more other antitumor agents;
(ii) the antitumor agent comprising, as an active ingredient, an anti-PD-1 antibody, which is used so as to be administered to a cancer patient in combination with futibatinib or a pharmaceutically acceptable salt thereof and at least one or more other antitumor agents; and
(iii) the antitumor agent comprising, as an active ingredient, at least one or more other antitumor agents, which is used so as to be administered to a cancer patient in combination with futibatinib or a pharmaceutically acceptable salt thereof and an anti-PD-1 antibody.

Further preferably, the antitumor agent includes an antitumor agent of any one of the following (i) to (iii), the antitumor agent not comprising, as an active ingredient, pembrolizumab:
(i) the antitumor agent comprising, as an active ingredient, futibatinib or a pharmaceutically acceptable salt thereof, which is used so as to be administered to a cancer patient in combination with zimberelimab and at least one or more other antitumor agents;
(ii) the antitumor agent comprising, as an active ingredient, zimberelimab, which is used so as to be administered to a cancer patient in combination with futibatinib or a pharmaceutically acceptable salt thereof and at least one or more other antitumor agents; and
(iii) the antitumor agent comprising, as an active ingredient, at least one or more other antitumor agents, which is used so as to be administered to a cancer patient in combination with futibatinib or a pharmaceutically acceptable salt thereof and zimberelimab.

The administration schedule of various active ingredients in the present invention can be appropriately set as long as the effect of the present invention is exhibited, but it is preferable to repeat a 21-day or 28-day administration cycle once or twice or more.

In the present invention, the administration schedule of futibatinib or a salt thereof is preferably administration once a day on each day of a 21-day or 28-day administration cycle.

In the present invention, the administration schedule of the immune checkpoint inhibitor can be appropriately set, but it is preferable to administer the immune checkpoint inhibitor at an administration frequency of once a day to once every four weeks. In the case of zimberelimab, for example, the administration frequency is preferably once every three weeks or once every two weeks. Specifically, administration once in a 21-day administration cycle or administration twice in a 28-day administration cycle.

In the present invention, the administration schedule of other antitumor agent can be appropriately set, but in a 21-day administration cycle, administration for 1 to 5 days is preferable, and administration for 1, 2, 3, 4, or 5 days is more preferable. In another embodiment of the present invention, as the administration schedule of other antitumor agent, for example, in a 28-day administration cycle, administration for 1 to 5 days is preferable, and administration for 1, 2, 3, 4, or 5 days is more preferable.

Examples of a preferable dose per day on the day of administration of futibatinib or a salt thereof include 4 mg to 160 mg, 4 mg to 24 mg, 12 to 24 mg, 16 to 24 mg, 20 mg, and the like. More specifically, the preferred number of administrations and dose per day on the day of administration include once a day, 4 mg/dose, 8 mg/dose, 12 mg/dose, 16 mg/dose, 20 mg/dose, and the like. In another embodiment, the preferred number of administrations and dose per day on the day of administration include once a day, 8 mg/dose, 12 mg/dose, 16 mg/dose, 20 mg/dose. In another embodiment, the preferred number of administrations and dose per day on the day of administration include once a day, 12 mg/dose, 16 mg/dose, 20 mg/dose. In another embodiment, the preferred number of administrations and dose per day on the day of administration include once a day and 20 mg/dose.

The present invention includes, in a certain embodiment, that the dose of futibatinib or a salt thereof is more than 20 mg/dose once a day in the case of cancer types (e.g., brain tumors) for which a stronger effect is desired.

In the present invention, the dose per day on the day of administration of the immune checkpoint inhibitor is appropriately set according to the drug, but in the case of nivolumab, the preferred dose per day on the day of administration includes 40 to 480 mg/dose, 80 mg/dose, 240 mg/dose, 360 mg/dose, or 480 mg/dose. Further, the administration interval of nivolumab is preferably an interval of 2 to 4 weeks, more preferably an interval of 2 to 3 weeks, and further preferably an interval of 2 weeks. In the case of zimberelimab, the preferred dose per day on the day of administration includes 240 to 480 mg/dose, 240 mg/dose, 360 mg/dose, 480 mg/dose, is preferably 240 mg/dose, or 360 mg/dose, and more preferably 360 mg/dose. Further, the administration interval of zimberelimab is preferably an interval of 2 to 4 weeks, more preferably an interval of 2 weeks or an interval of 3 weeks, and further preferably an interval of 2 weeks.

As one embodiment of the present invention, the administration interval and dose of zimberelimab are administration of 360 mg/dose once every 3 weeks.

As one embodiment of the present invention, the administration interval and dose of zimberelimab are administration of 240 mg/dose once every 2 weeks.

In the present invention, "cancer" or "tumor" refers to the physiological conditions of a mammal characterized by unregulated cell growth. "Cancer" and "tumor" have the same meaning in the present specification and are used interchangeably. Cancers include solid and hematologic cancers. Examples include, but are not limited to, carcinoma, lymphoma, leukemia, blastoma, sarcoma, and borderline malignancy (carcinoid).

Examples of cancers targeted by the combination method of the present invention include, but are not limited to the presence or absence of an abnormality in the FGFR pathway, head and neck cancer, digestive organ cancer (esophageal cancer, gastric cancer, duodenum cancer, liver cancer (hepatocellular cancer), biliary tract cancer (e.g., gallbladder and cholangiocarcinoma), pancreatic cancer, small intestine cancer, large intestine cancer (e.g., colorectal cancer, colon cancer, and rectal cancer), etc.), lung cancer (e.g., non-small cell lung cancer and small cell lung cancer), mesothelioma (e.g., malignant pleural mesothelioma, peritoneal mesothelioma, pericardial mesothelioma, and testicular mesothelioma), breast cancer, genital cancer (ovarian cancer, uterine cancer (e.g., cervical cancer, uterine body cancer, and endometrial cancer), etc.), renal cancer, bladder cancer, urothelial cancer, prostate cancer, testicular tumor, skin cancer (e.g., malignant melanoma and epidermal cancer), blood cancer (e.g., multiple myeloma, malignant lymphoma, leukemia, etc.), bone and soft tissue tumor, rhabdomyosarcoma, brain tumor, malignant schwannoma, neuroendocrine tumor, thyroid cancer, and the like. Note that, the cancers mentioned herein include not only the primary lesion, but also cancer metastasized to other organs (e.g., liver). The antitumor agent of the present invention may also be used in postoperative adjuvant chemotherapy performed in order to prevent recurrence after surgical removal of the tumor, or may be used in preoperative adjuvant chemotherapy performed in order to surgically remove the tumor.

In the present invention, preferable cancers include lung cancer, esophageal cancer, gastric cancer, duodenum cancer, liver cancer, hepatocellular cancer, biliary tract cancer, pancreatic cancer, large bowel cancer, breast cancer, uterine cancer, ovarian cancer, renal cancer, bladder cancer, prostate cancer, testicular tumor, thyroid cancer, bone or soft tissue tumor, leukemia, malignant lymphoma, multiple myeloma, head and neck cancer, brain tumor, mesothelioma, skin cancer, and cancer of unknown primary site, solid cancer is preferable, pancreatic cancer, lung cancer, esophageal cancer, biliary tract cancer, or head and neck cancer is more preferable, pancreatic cancer, lung cancer, esophageal cancer, biliary tract cancer, or head and neck cancer is further preferable, pancreatic cancer, non-small cell lung cancer, esophageal cancer, biliary tract cancer, or head and neck cancer is still more preferable, pancreatic cancer, non-small cell lung cancer, esophageal cancer, biliary tract cancer, or head and neck cancer, which has not been previously treated for advanced cancer or has been previously treated with one line of chemotherapy, is even more preferable, and pancreatic cancer, non-small cell lung cancer, esophageal cancer, biliary tract cancer, or head and neck cancer, which has not been previously treated for advanced cancer, is particularly preferable.

As one embodiment of the present invention, a subject is a patient with esophageal cancer who has not been previously treated for advanced cancer.

As one embodiment of the present invention, a subject is a patient with esophageal cancer who has not been previously treated for advanced cancer or who has been previously treated with one line of chemotherapy.

As one embodiment of the present invention, a subject is a patient with head and neck cancer who has not been previously treated for advanced cancer.

As one embodiment of the present invention, a subject is a patient with non-small cell lung cancer who has not been previously treated for advanced cancer.

As one embodiment of the present invention, a subject is a patient with biliary tract cancer who has not been previously treated for advanced cancer.

As one embodiment of the present invention, a subject is a patient with pancreatic cancer who has not been previously treated for advanced cancer.

Suitable antitumor agents in the present invention include, but are not limited to, the following.

An antitumor agent of any one of the following (i) to (iv) is preferable:
(i) the antitumor agent comprising, as an active ingredient, futibatinib or a pharmaceutically acceptable salt thereof, which is used so as to be administered to a patient with esophageal cancer in combination with zimberelimab, cisplatin, and 5-fluorouracil;
(ii) the antitumor agent comprising, as an active ingredient, zimberelimab, which is used so as to be administered to a patient with esophageal cancer in combination with futibatinib or a pharmaceutically acceptable salt thereof, cisplatin, and 5-fluorouracil;
(iii) the antitumor agent comprising, as an active ingredient, cisplatin, which is used so as to be administered to a patient with esophageal cancer in combination with futibatinib or a pharmaceutically acceptable salt thereof, zimberelimab, and 5-fluorouracil; and
(iv) the antitumor agent comprising, as an active ingredient, 5-fluorouracil, which is used so as to be administered to a patient with esophageal cancer in combination with futibatinib or a pharmaceutically acceptable salt thereof, zimberelimab, and cisplatin.

The antitumor agent of any one of the above (i) to (iv), in which a 21-day administration cycle in which 12 mg/dose, 16 mg/dose, or 20 mg/dose of futibatinib is administered daily once a day, 360 mg/dose of zimberelimab is administered once, 80 mg/m²/dose of cisplatin is administered once, and 800 mg/m²/day of 5-fluorouracil is administered daily for 5 days is repeated once or twice or more, is further preferable, and the antitumor agent of any one of the above (i) to (iv), in which a 21-day administration cycle in which 12 mg/dose, 16 mg/dose, or 20 mg/dose of futibatinib is administered daily once a day, 360 mg/dose of zimberelimab is administered once on Day 1, 80 mg/m²/dose of cisplatin is administered once on Day 1, and 800 mg/m²/day of 5-fluorouracil is administered daily on Days 1 to 5 is repeated once or twice or more, is particularly preferable.

One embodiment of the present invention includes the antitumor agent of any one of the above (i) to (iv), in which a 21-day administration cycle in which 12 mg/dose of futibatinib is administered daily once a day, 360 mg/dose of zimberelimab is administered once on Day 1, 80 mg/m²/dose of cisplatin is administered once on Day 1, and 800 mg/m²/day of 5-fluorouracil is administered daily on Days 1 to 5 is repeated once or twice or more.

In the present invention, "mg/m²/dose" refers to a dose (mg) per body surface area (m²) per administration.

One embodiment of the present invention includes the antitumor agent of any one of the above (i) to (iv), in which a 21-day administration cycle in which 16 mg/dose of futibatinib is administered daily once a day, 360 mg/dose of zimberelimab is administered once on Day 1, 80 mg/m²/dose of cisplatin is administered once on Day 1, and 800 mg/m²/day of 5-fluorouracil is administered daily on Days 1 to 5 is repeated once or twice or more.

One embodiment of the present invention includes the antitumor agent of any one of the above (i) to (iv), in which a 21-day administration cycle in which 20 mg/dose of futibatinib is administered daily once a day, 360 mg/dose of zimberelimab is administered once on Day 1, 80 mg/m²/dose of cisplatin is administered once on Day 1, and 800 mg/m²/day of 5-fluorouracil is administered daily on Days 1 to 5 is repeated once or twice or more.

An antitumor agent of any one of the following (i) to (iii) is preferable:
(i) the antitumor agent comprising, as an active ingredient, futibatinib or a pharmaceutically acceptable salt thereof, which is used so as to be administered to a patient with esophageal cancer in combination with zimberelimab and domvanalimab;
(ii) the antitumor agent comprising, as an active ingredient, zimberelimab, which is used so as to be administered to a patient with esophageal cancer in combination with futibatinib or a pharmaceutically acceptable salt thereof, and domvanalimab; and
(iii) the antitumor agent comprising, as an active ingredient, domvanalimab, which is used so as to be administered to a patient with esophageal cancer in combination with futibatinib or a pharmaceutically acceptable salt thereof, and zimberelimab.

The antitumor agent of any one of the above (i) to (iii), in which a 21-day administration cycle in which 12 mg/dose, 16 mg/dose, or 20 mg/dose of futibatinib is administered daily once a day, 360 mg/dose of zimberelimab is administered once, and 1200 mg/dose of domvanalimab is administered once is repeated once or twice or more, is further preferable, and the antitumor agent of any one of the above (i) to (iii), in which a 21-day administration cycle in which 12 mg/dose, 16 mg/dose, or 20 mg/dose of futibatinib is administered daily once a day, 360 mg/dose of zimberelimab is administered once on Day 1, and 1200 mg/dose of domvanalimab is administered once on Day 1 is repeated once or twice or more, is particularly preferable.

One embodiment of the present invention includes the antitumor agent of any one of the above (i) to (iii), in which a 21-day administration cycle in which 12 mg/dose of futibatinib is administered daily once a day, 360 mg/dose of zimberelimab is administered once on Day 1, and 1200 mg/dose of domvanalimab is administered once on Day 1 is repeated once or twice or more.

One embodiment of the present invention includes the antitumor agent of any one of the above (i) to (iii), in which a 21-day administration cycle in which 16 mg/dose of futibatinib is administered daily once a day, 360 mg/dose of zimberelimab is administered once on Day 1, and 1200 mg/dose of domvanalimab is administered once on Day 1 is repeated once or twice or more.

One embodiment of the present invention includes the antitumor agent of any one of the above (i) to (iii), in which a 21-day administration cycle in which 20 mg/dose of futibatinib is administered daily once a day, 360 mg/dose of zimberelimab is administered once on Day 1, and 1200 mg/dose of domvanalimab is administered once on Day 1 is repeated once or twice or more.

An antitumor agent of any one of the following (i) to (iv) is preferable:
(i) the antitumor agent comprising, as an active ingredient, futibatinib or a pharmaceutically acceptable salt thereof, which is used so as to be administered to a patient with head and neck cancer in combination with zimberelimab, 5-fluorouracil, and carboplatin or cisplatin;
(ii) the antitumor agent comprising, as an active ingredient, zimberelimab, which is used so as to be administered to a patient with head and neck cancer in combination with futibatinib or a pharmaceutically acceptable salt thereof, 5-fluorouracil, and carboplatin or cisplatin;
(iii) the antitumor agent comprising, as an active ingredient, 5-fluorouracil, which is used so as to be administered to a patient with head and neck cancer in combination with futibatinib or a pharmaceutically acceptable salt thereof, zimberelimab, and carboplatin or cisplatin; and
(iv) the antitumor agent comprising, as an active ingredient, carboplatin or cisplatin, which is used so as to be administered to a patient with head and neck cancer in combination with futibatinib or a pharmaceutically acceptable salt thereof, zimberelimab, and 5-fluorouracil.

The antitumor agent of any one of the above (i) to (iv), in which a 21-day administration cycle in which 12 mg/dose, 16 mg/dose, or 20 mg/dose of futibatinib is administered daily once a day, 360 mg/dose of zimberelimab is administered once, AUC 5 carboplatin or 100 mg/m²/dose of cisplatin is administered once, and 1000 mg/m²/day of 5-fluorouracil is administered daily for 4 days is repeated once or twice or more, is further preferable, and the antitumor agent of any one of the above (i) to (iv), in which a 21-day administration cycle in which 12 mg/dose, 16 mg/dose, or 20 mg/dose of futibatinib is administered daily once a day, 360 mg/dose of zimberelimab is administered once on Day 1, AUC 5 carboplatin or 100 mg/m²/dose of cisplatin is administered once on Day 1, and 1000 mg/m²/day of 5-fluorouracil is administered daily on Days 1 to 4 is repeated once or twice or more, is particularly preferable.

One embodiment of the present invention includes the antitumor agent of any one of the above (i) to (iv), in which a 21-day administration cycle in which 12 mg/dose of futibatinib is administered daily once a day, 360 mg/dose of zimberelimab is administered once on Day 1, AUC 5 carboplatin or 100 mg/m²/dose of cisplatin is administered once on Day 1, and 1000 mg/m²/day of 5-fluorouracil is administered daily on Days 1 to 4 is repeated once or twice or more.

One embodiment of the present invention includes the antitumor agent of any one of the above (i) to (iv), in which a 21-day administration cycle in which 16 mg/dose of futibatinib is administered daily once a day, 360 mg/dose of zimberelimab is administered once on Day 1, AUC 5 carboplatin or 100 mg/m²/dose of cisplatin is administered once on Day 1, and 1000 mg/m²/day of 5-fluorouracil is administered daily on Days 1 to 4 is repeated once or twice or more.

One embodiment of the present invention includes the antitumor agent of any one of the above (i) to (iv), in which a 21-day administration cycle in which 20 mg/dose of futibatinib is administered daily once a day, 360 mg/dose of zimberelimab is administered once on Day 1, AUC 5 carboplatin or 100 mg/m²/dose of cisplatin is administered once on Day 1, and 1000 mg/m²/day of 5-fluorouracil is administered daily on Days 1 to 4 is repeated once or twice or more.

An antitumor agent of any one of the following (i) to (iii) is preferable:
(i) the antitumor agent comprising, as an active ingredient, futibatinib or a pharmaceutically acceptable salt thereof, which is used so as to be administered to a patient with head and neck cancer in combination with zimberelimab and domvanalimab;
(ii) the antitumor agent comprising, as an active ingredient, zimberelimab, which is used so as to be administered to a patient with head and neck cancer in combination with futibatinib or a pharmaceutically acceptable salt thereof, and domvanalimab; and
(iii) the antitumor agent comprising, as an active ingredient, domvanalimab, which is used so as to be administered to a patient with head and neck cancer in combination with futibatinib or a pharmaceutically acceptable salt thereof, and zimberelimab.

The antitumor agent of any one of the above (i) to (iii), in which a 21-day administration cycle in which 12 mg/dose, 16 mg/dose, or 20 mg/dose of futibatinib is administered daily once a day, 360 mg/dose of zimberelimab is administered once, and 1200 mg/dose of domvanalimab is administered once is repeated once or twice or more, is further preferable, and the antitumor agent of any one of the above (i) to (iii), in which a 21-day administration cycle in which 12 mg/dose, 16 mg/dose, or 20 mg/dose of futibatinib is administered daily once a day, 360 mg/dose of zimberelimab is administered once on Day 1, and 1200 mg/dose of domvanalimab is administered once on Day 1 is repeated once or twice or more, is particularly preferable.

One embodiment of the present invention includes the antitumor agent of any one of the above (i) to (iii), in which a 21-day administration cycle in which 12 mg/dose of futibatinib is administered daily once a day, 360 mg/dose of zimberelimab is administered once on Day 1, and 1200 mg/dose of domvanalimab is administered once on Day 1 is repeated once or twice or more.

One embodiment of the present invention includes the antitumor agent of any one of the above (i) to (iii), in which a 21-day administration cycle in which 16 mg/dose of futibatinib is administered daily once a day, 360 mg/dose of zimberelimab is administered once on Day 1, and 1200 mg/dose of domvanalimab is administered once on Day 1 is repeated once or twice or more.

One embodiment of the present invention includes the antitumor agent of any one of the above (i) to (iii), in which a 21-day administration cycle in which 20 mg/dose of futibatinib is administered daily once a day, 360 mg/dose of zimberelimab is administered once on Day 1, and 1200 mg/dose of domvanalimab is administered once on Day 1 is repeated once or twice or more.

An antitumor agent of any one of the following (i) to (iv) is preferable:
(i) the antitumor agent comprising, as an active ingredient, futibatinib or a pharmaceutically acceptable salt thereof, which is used so as to be administered to a patient with non-small cell lung cancer in combination with zimberelimab, albumin-bound paclitaxel, and carboplatin;
(ii) the antitumor agent comprising, as an active ingredient, zimberelimab, which is used so as to be administered to a patient with non-small cell lung cancer in combination with futibatinib or a pharmaceutically acceptable salt thereof, albumin-bound paclitaxel, and carboplatin;
(iii) the antitumor agent comprising, as an active ingredient, albumin-bound paclitaxel, which is used so as to be administered to a patient with non-small cell lung cancer in combination with futibatinib or a pharmaceutically acceptable salt thereof, zimberelimab, and carboplatin; and
(iv) the antitumor agent comprising, as an active ingredient, carboplatin, which is used so as to be administered to a patient with non-small cell lung cancer in combination with futibatinib or a pharmaceutically acceptable salt thereof, zimberelimab, and albumin-bound paclitaxel.

The antitumor agent of any one of the above (i) to (iv), in which a 21-day administration cycle in which 12 mg/dose, 16 mg/dose, or 20 mg/dose of futibatinib is administered daily once a day, 360 mg/dose of zimberelimab is administered once, 100 mg/m²/dose of albumin-bound paclitaxel is administered three times, and AUC 6 carboplatin is administered once is repeated once or twice or more, is further preferable, and the antitumor agent of any one of the above (i) to (iv), in which a 21-day administration cycle in which 12 mg/dose, 16 mg/dose, or 20 mg/dose of futibatinib is administered daily once a day, 360 mg/dose of zimberelimab is administered on Day 1, 100 mg/m²/dose of albumin-bound paclitaxel is administered once on Day 1, Day 8, and Day 15, and AUC 6 carboplatin is administered on Day 1 is repeated once or twice or more, is particularly preferable.

One embodiment of the present invention includes the antitumor agent of any one of the above (i) to (iv), in which a 21-day administration cycle in which 12 mg/dose of futibatinib is administered daily once a day, 360 mg/dose of zimberelimab is administered on Day 1, 100 mg/m²/dose of albumin-bound paclitaxel is administered once on Day 1, Day 8, and Day 15, and AUC 6 carboplatin is administered on Day 1 is repeated once or twice or more.

One embodiment of the present invention includes the antitumor agent of any one of the above (i) to (iv), in which a 21-day administration cycle in which 16 mg/dose of futibatinib is administered daily once a day, 360 mg/dose of zimberelimab is administered on Day 1, 100 mg/m²/dose of albumin-bound paclitaxel is administered once on Day 1, Day 8, and Day 15, and AUC 6 carboplatin is administered on Day 1 is repeated once or twice or more.

One embodiment of the present invention includes the antitumor agent of any one of the above (i) to (iv), in which a 21-day administration cycle in which 20 mg/dose of futibatinib is administered daily once a day, 360 mg/dose of zimberelimab is administered on Day 1, 100 mg/m²/dose of albumin-bound paclitaxel is administered once on Day 1, Day 8, and Day 15, and AUC 6 carboplatin is administered on Day 1 is repeated once or twice or more.

An antitumor agent of any one of the following (i) to (iv) is preferable:
(i) the antitumor agent comprising, as an active ingredient, futibatinib or a pharmaceutically acceptable salt thereof, which is used so as to be administered to a patient with biliary tract cancer in combination with zimberelimab, cisplatin, and gemcitabine;
(ii) the antitumor agent comprising, as an active ingredient, zimberelimab, which is used so as to be administered to a patient with biliary tract cancer in combination with futibatinib or a pharmaceutically acceptable salt thereof, cisplatin, and gemcitabine;
(iii) the antitumor agent comprising, as an active ingredient, gemcitabine, which is used so as to be administered to a patient with biliary tract cancer in combination with futibatinib or a pharmaceutically acceptable salt thereof, zimberelimab, and cisplatin; and
(iv) the antitumor agent comprising, as an active ingredient, cisplatin, which is used so as to be administered to a patient with biliary tract cancer in combination with futibatinib or a pharmaceutically acceptable salt thereof, zimberelimab, and gemcitabine.

The antitumor agent of any one of the above (i) to (iv), in which a 21-day administration cycle in which 12 mg/dose, 16 mg/dose, or 20 mg/dose of futibatinib is administered daily once a day, 360 mg/dose of zimberelimab is administered once, 100 mg/m²/dose of albumin-bound paclitaxel is administered three times, and AUC 6 carboplatin is administered once is repeated once or twice or more, is further preferable, and the antitumor agent of any one of the above (i) to (iv), in which a 21-day administration cycle in which 12 mg/dose, 16 mg/dose, or 20 mg/dose of futibatinib is administered daily once a day, 360 mg/dose of zimberelimab is administered on Day 1, 100 mg/m²/dose of albumin-bound paclitaxel is administered once on Day 1, Day 8, and Day 15, and AUC 6 carboplatin is administered on Day 1 is repeated once or twice or more, is particularly preferable.

One embodiment of the present invention includes the antitumor agent of any one of the above (i) to (iv), in which a 21-day administration cycle in which 12 mg/dose of futibatinib is administered daily once a day, 360 mg/dose of zimberelimab is administered on Day 1, 100 mg/m²/dose of albumin-bound paclitaxel is administered once on Day 1, Day 8, and Day 15, and AUC 6 carboplatin is administered on Day 1 is repeated once or twice or more.

One embodiment of the present invention includes the antitumor agent of any one of the above (i) to (iv), in which a 21-day administration cycle in which 16 mg/dose of futibatinib is administered daily once a day, 360 mg/dose of zimberelimab is administered on Day 1, 100 mg/m²/dose of albumin-bound paclitaxel is administered once on Day 1, Day 8, and Day 15, and AUC 6 carboplatin is administered on Day 1 is repeated once or twice or more.

One embodiment of the present invention includes the antitumor agent of any one of the above (i) to (iv), in which a 21-day administration cycle in which 20 mg/dose of futibatinib is administered daily once a day, 360 mg/dose of zimberelimab is administered on Day 1, 100 mg/m²/dose of albumin-bound paclitaxel is administered once on Day 1, Day 8, and Day 15, and AUC 6 carboplatin is administered on Day 1 is repeated once or twice or more.

An antitumor agent of any one of the following (i) to (iv) is preferable:
(i) the antitumor agent comprising, as an active ingredient, futibatinib or a pharmaceutically acceptable salt thereof, which is used so as to be administered to a patient with pancreatic cancer in combination with zimberelimab, albumin-bound paclitaxel, and gemcitabine;
(ii) the antitumor agent comprising, as an active ingredient, zimberelimab, which is used so as to be administered to a patient with pancreatic cancer in combination with futibatinib or a pharmaceutically acceptable salt thereof, albumin-bound paclitaxel, and gemcitabine;
(iii) the antitumor agent comprising, as an active ingredient, gemcitabine, which is used so as to be administered to a patient with pancreatic cancer in combination with futibatinib or a pharmaceutically acceptable salt thereof, zimberelimab, and albumin-bound paclitaxel; and
(iv) the antitumor agent comprising, as an active ingredient, albumin-bound paclitaxel, which is used so as to be administered to a patient with pancreatic cancer in combination with futibatinib or a pharmaceutically acceptable salt thereof, zimberelimab, and gemcitabine.

The antitumor agent of any one of the above (i) to (iv), in which a 28-day administration cycle in which 12 mg/dose, 16 mg/dose, or 20 mg/dose of futibatinib is administered daily once a day, 240 mg/dose of zimberelimab is administered twice, 125 mg/m²/dose of albumin-bound paclitaxel is administered three times, and 1000 mg/m²/dose of gemcitabine is administered three times is repeated once or twice or more, is further preferable, and the antitumor agent of any one of the above (i) to (iv), in which a 28-day administration cycle in which 12 mg/dose, 16 mg/dose, or 20 mg/dose of futibatinib is administered daily once a day, 240 mg/dose of zimberelimab is administered on Day 1 and Day 15, 125 mg/m²/dose of albumin-bound paclitaxel is administered once on Day 1, Day 8, and Day 15, and 1000 mg/m²/dose of gemcitabine is administered once on Day 1, Day 8, and Day 15 is repeated once or twice or more, is particularly preferable.

One embodiment of the present invention includes the antitumor agent of any one of the above (i) to (iv), in which a 28-day administration cycle in which 12 mg/dose of futibatinib is administered daily once a day, 240 mg/dose of zimberelimab is administered on Day 1 and Day 15, 125 mg/m²/dose of albumin-bound paclitaxel is administered once on Day 1, Day 8, and Day 15, and 1000 mg/m²/dose of gemcitabine is administered once on Day 1, Day 8, and Day 15 is repeated once or twice or more.

One embodiment of the present invention includes the antitumor agent of any one of the above (i) to (iv), in which a 28-day administration cycle in which 16 mg/dose of futibatinib is administered daily once a day, 240 mg/dose of zimberelimab is administered on Day 1 and Day 15, 125 mg/m²/dose of albumin-bound paclitaxel is administered once on Day 1, Day 8, and Day 15, and 1000 mg/m²/dose of gemcitabine is administered once on Day 1, Day 8, and Day 15 is repeated once or twice or more.

One embodiment of the present invention includes the antitumor agent of any one of the above (i) to (iv), in which a 28-day administration cycle in which 20 mg/dose of futibatinib is administered daily once a day, 240 mg/dose of zimberelimab is administered on Day 1 and Day 15, 125 mg/m²/dose of albumin-bound paclitaxel is administered once on Day 1, Day 8, and Day 15, and 1000 mg/m²/dose of gemcitabine is administered once on Day 1, Day 8, and Day 15 is repeated once or twice or more.

As used in the present specification, the term "combination (therapy)" is intended to define a therapy that includes the use of a combination of two or more compounds/drugs (as defined above). Thus, "combination (therapy)", "combination", and the use of compounds/drugs "in combination" in the present application may mean compounds/drugs administered as part of the same overall therapeutic regimen. The dose of each of the two or more compounds/drugs may be different: each may be administered simultaneously or at different times. Therefore, it is understood that the compounds/drugs of the combination may be administered sequentially (e.g., before or after) or simultaneously, either in the same pharmaceutical formulation (i.e., together) or in different pharmaceutical formulations (i.e., separately). The same formulation is simultaneously a single formulation, but different pharmaceutical formulations are non-integral.

The administration form of the antitumor agent of the present invention is not particularly limited, and can be appropriately selected depending on the therapeutic purpose, and specific examples thereof include oral preparations (tablets, coated tablets, powders, granules, capsules, solutions, etc.), injections, suppositories, patches, ointments, and the like. In the case of futibatinib or a salt thereof, oral preparations are preferable. In the case of an immune checkpoint inhibitor and other antitumor agent, the above-mentioned administration forms are exemplified, and injections are preferable. In the case of zimberelimab, domvanalimab, carboplatin, cisplatin, 5-fluorouracil, albumin-bound paclitaxel, or gemcitabine, injections are preferable.

According to the antitumor agent of the present invention, an immune checkpoint inhibitor and futibatinib or a salt thereof, which are active ingredients, may be directly used as an antitumor agent, and depending on the administration form thereof, a pharmaceutically acceptable carrier may be used to prepare a pharmaceutical composition by a generally known method. Examples of such carriers include various carriers commonly used in general drugs, such as excipients, binders, disintegrants, lubricants, diluents, solubilizing agents, suspending agents, isotonizing agents, pH adjusters, buffers, stabilizers, coloring agents, flavoring agents, odor masking agent, and the like.

The antitumor agent of the present invention may be formulated into a plurality of dosage forms, or may be formulated into a single dosage form, based on the administration form and administration schedule of each active ingredient. Further, formulations may be produced and sold in a single package suitable for combined administration, or formulations may be produced and sold in separate packages. The same applies to the embodiments of the pharmaceutical composition. Accordingly, the "pharmaceutical composition comprising an immune checkpoint inhibitor and futibatinib or a salt thereof as active ingredients" includes a pharmaceutical composition prepared by separately formulating the active ingredients in a plurality of dosage forms, and a pharmaceutical composition prepared by formulating the active ingredients in a single dosage form. The pharmaceutical composition prepared by separately formulating the active ingredients in a plurality of dosage forms includes a pharmaceutical composition prepared by formulating formulations in a single package suitable for combined administration, and a pharmaceutical composition prepared by formulating formulations in separate packages.

The present invention relates to a kit preparation comprising an antitumor agent comprising futibatinib or a salt thereof, and an instruction manual stating that futibatinib or a salt thereof is administered to cancer patients in combination with an immune checkpoint inhibitor. The "instruction manual" as used herein may be one stating the above-mentioned dose, and regardless of whether it is legally binding, an instruction manual that recommends the above-mentioned dose is preferable. Specific examples include package inserts, brochures, and the like. The kit preparation comprising an instruction manual may be one in which the instruction manual is printed or attached to the package of the kit preparation, or may be one in which the instruction manual is enclosed together with an antitumor agent in the package of the kit preparation.

The treatment in the present invention includes procedures performed for the purpose of curing or ameliorating diseases, or for the purpose of suppressing disease progression or relapse or alleviating the symptoms. The treatment includes administration of drugs before or after surgical procedure, or administration of drugs during, before, or after radiation therapy.

The antitumor agent of the present invention can be used for the treatment of cancer. The "antitumor effect" when referring to a cancer patient who receives treatment with a therapeutic regimen, such as the combination therapy described in the present specification, means at least one evaluation of, for example, PFS (progression-free survival), DCR (disease control rate), DOR (duration of response), OS (overall survival), ORR (objective response rate), DCR (disease control rate), TTR (time to first response), PROs (patient-reported outcomes), and the like. In one embodiment, tumor evaluation for the combination therapy described in the present specification for solid cancers is based on the RECIST 1.1 criteria (Response Evaluation Criteria in Solid Cancers), and the antitumor effect is expressed by SD (stable disease), PR (partial response), CR (complete response), and PD (progressive disease). Tumor evaluation of brain tumors can be carried out by standard brain tumor MRI, including pre- and post-enhancement MRI, using a Gd-MRI (gadolinium (Gd)) chelate contrast agent.

### Examples

### Example Phase 1a/b Clinical Trial on AB122 Combination Therapy in Patients with Advanced Solid Cancer

### Purpose and Endpoint:

**Table 1**

| **Purpose** | **Endpoint** |
|---|---|
| **Primary** | |
| **Purpose** | **Endpoint** |
| **Phase 1a** | **Phase 1a (all cohorts except for cohorts D4 and D-5)** |
| - To evaluate tolerability and safety of AB122 combination therapy in patients with advanced solid cancer | |
| | - DL Tin Cycle 1 |
| | **Phase 1a (cohorts D4 and D-5)** |
| **Phase 1b** | - Adverse events and expression ratio of adverse events associated with treatment |
| - To evaluate antitumor effect of AB122 combination therapy | |
| | **Phase 1b** |
| | - ORR based on RECIST v1.1 |

| **Secondary** | |
|---|---|
| **Phase 1a** | **Phase 1a** |
| - To evaluate general safety and tolerability of AB122 combination therapy | - Adverse events, adverse events associated with treatment, and laboratory abnormalities |
| - To clarify PK profile of AB122 combination therapy (each component) in patients with advanced solid cancer | |
| | - Dose intensity |
| | - PK parameter |
| - To clarify immunogenicity of biological component of AB122 combination therapy | - Number of patients expressing ADA and proportion |
| - To examine correlation between predictive biomarker and clinical effect | - Correlation between clinical effect and predictive biomarker |
| - To examine pharmacodynamic action of AB122 combination therapy | - Change in pharmacodynamic parameter |
| | - ORR, PFS, DCR, and DoR based on RECISTv1.1 and iRECIST |
| - To evaluate antitumor effect of AB122 combination therapy | |
| | - OS |

| **Purpose** | **Endpoint** |
|---|---|
| **Phase 1b** | **Phase 1b** |
| - To evaluate safety of AB122 combination therapy | - Adverse events, adverse events associated with treatment, and laboratory abnormalities |
| - To clarify PK profile of AB122 combination therapy (each component) in patients with advanced solid cancer | |
| | - PK parameter |
| | - Number of patients expressing ADA and proportion |
| - To clarify immunogenicity of biological component of AB122 combination therapy | |
| | - Correlation between clinical effect and predictive biomarker |
| - To examine correlation between predictive biomarker and clinical effect | |
| | - Change in pharmacodynamic parameter |
| - To examine pharmacodynamic action of AB122 combination therapy | - PFS, DCR, and DoR |
| | - OS |
| - To evaluate antitumor effect of AB122 combination therapy | |

### Method:

This trial is Phase 1, non-randomized, open-label, multi-center, platform study to evaluate the tolerability and safety of AB122 in patients with malignant tumors.

This trial is composed of two phases (Phase 1a and Phase 1b). In Phase 1a, the purpose is to evaluate the safety and tolerability of a combination therapy based on AB122 in patients with advanced solid cancer, determine the recommended dose (RD) for each regimen, and evaluate the efficacy at RD. In Phase 1b, the purpose is to add patients to the appropriate cohort and evaluate the safety and efficacy of a treatment method based on AB122.

**Table 2**

| Summary of Trial Target Population and Trial Treatment in Each Cohort | | |
|---|---|---|
| **Cohort** | **Trial target population** | **Trial treatment** |
| D-2 | Patients with esophageal cancer who has not been previously treated for advanced cancer | Combination therapy of AB122 (Q3W), futibatinib, and chemotherapy |
| D-3 | Patients with esophageal cancer who has not been previously treated for advanced cancer or who has been previously treated with one line of chemotherapy | Combination therapy of AB122 (Q3W), AB154, and futibatinib |
| D-4 | Patients with head and neck cancer who has not been previously treated for advanced cancer | Combination therapy of AB122 (Q3W), futibatinib, and chemotherapy |
| D-5 | Patients with squamous non-small cell lung cancer who has not been previously treated for advanced cancer | Combination therapy of AB122 (Q3W), AB154, and futibatinib |
| D-6 | Patients with biliary tract cancer who has not been previously treated for advanced cancer | Combination therapy of AB122 (Q3W), futibatinib, and chemotherapy |
| D-7 | Patients with pancreatic cancer who has not been previously treated for advanced cancer | Combination therapy of AB122 (Q3W), futibatinib, and chemotherapy |
| D-8 | Combination therapy of AB122 (Q3W), futibatinib, and chemotherapy | Combination therapy of AB122 (Q2W), futibatinib, and chemotherapy |

| | | |
|---|---|---|
| **Q2W onoe every two weeks, Q3W: once every three weeks** | | |

### Phase 1a

When the tumor reduction effect based on RECIST v1.1 is determined in the first five patients, the registration may be stopped in a case where the probability that the number of required response cases will be observed in other five patients according to the binomial distribution of the response rate is 20% or less.

Dose-limiting toxicity (DLT): The following adverse events associated with study drug that occurred in Cycle 1 are defined as DLT. The following grades are in accordance with National Cancer Institute (NCI) Common Terminology Criteria for Adverse Events (CTCAE) v 5.0.

The investigator and the sponsor will determine whether or not laboratory abnormalities (except for the following hematotoxicity and non-hematotoxicity) and transient signs or symptoms correspond to DLT.
- Grade 4 neutropenia lasting more than 7 days
- Febrile neutropenia [an absolute neutrophil count (ANC) of less than 1000/mm³, and a body temperature of greater than 38.3°C at least once or persistently equal to or greater than 38°C for more than 1 hour]
- Grade 4 thrombocytopenia or Grade 3 thrombocytopenia with bleeding requiring blood transfusion
- Grade 4 anemia or Grade 3 anemia requiring blood transfusion
- Grade 3 or higher non-hematologic adverse events are considered DLT. Provided that, Grade 3 fatigue with a duration of 3 days or less, Grade 3 diarrhea, nausea or vomiting with or without the use of antiemetics or antidiarrheals based on standard of care, and Grade 3 rash without the use of corticosteroids or anti-inflammatory agents based on standard of care are excluded.
- Grade 2 or higher aspartate aminotransferase (AST) or alanine aminotransferase (ALT) increase with a total bilirubin increase of more than 2 times the upper limit of reference (ULN). Provided that, there are no initial findings of cholestasis [alkali phosphatase (ALP) increase to less than 2-fold of ULN] and no other reason to explain these increases.
- Toxicity associated with study drug that hinders end of Cycle 1 [defined as a period in which the number of oral doses of the study drug is 75% or more of the specified value and the whole doses of AB122 and AB154 (only cohort D-3) are administered] and hinders start of Cycle 2
- Hyperphosphatemia:
   Increase in serum phosphorus level of 10 mg/dL or more, or
   increase in serum phosphorus of 7 mg/dL or more lasting 7 days or more despite phosphate lowering therapy for 7 days

### Phase 1b

The Phase 1b part starts after tolerability and preliminary efficacy are determined based on the results of the Phase 1a part. The cohort to be expanded to Phase 1b part will be determined through consultation between the Data Monitoring Committee (DMC) and the sponsor.

### Target Number of Cases (Planned):

### Cohort D-2

The target number of cases is maximum 52 for Phase 1a and maximum 30 for Phase 1b.

### Cohort D-3

The target number of cases is maximum 52 for Phase 1a and maximum 30 for Phase 1b.

### Cohort D-4

The target number of cases is 40 for Phase 1a and maximum 30 for Phase 1b.

### Cohort D-5

The target number of cases is 40 for Phase 1a and maximum 30 for Phase 1b.

### Cohort D-6

The target number of cases is maximum 52 for Phase 1a and maximum 30 for Phase 1b.

### Cohort D-7

The target number of cases is maximum 52 for Phase 1a and maximum 30 for Phase 1b.

### Cohort D-8

The target number of cases is maximum 52 for Phase 1a and maximum 30 for Phase 1b.

### Diagnosis and Main Inclusion Criteria:

Patients with advanced solid cancer who are 18 years of age or older at the time of obtaining consent.

### Dose and Administration Method:

### Phase 1a

### Administration Method:

The administration method in each cohort is shown below.

**Table 3**

| Summary of Trial Treatment | | | | |
|---|---|---|---|---|
| **Cohort** | **Study drug** | **Starting dose** | **Administration interval** | **Administration route** |
| D-2-7 | AB122 | 360 mg/body | Q3W | IV |
| D-8 | AB122 | 240 mg/body | Q2W | IV |
| D-n | Futibatinib | Level 1: 20 mg/day | QD | Orally |
| D-2 | 5-Fluorouracil | 800 mg/m²/ day | Administered on Days 1 to 5 for 21 days in one cycle | IV |
| | Cisplatin | 80 mg/m² | Q3W | IV |
| D-3, D-5 | AB154 | 1200 mg/body | Q3W | IV |
| D-4 | 5-Fluorouracil | 1000 mg/m²/day | Administered on Days 1 to 4 for 21 days in one cycle | IV |
| | Carboplatin | AUC 5 | Q3W | IV |
| | Cisplatin | 100 mg/m² | Q3W | IV |
| D-6 | Carboplatin | AUC 6 | Q3W | IV |
| | Albumin-bound paclitaxel | 100 mg/m² | Weekly (Day 1, 8, 15) | IV |
| D-7 | Cisplatin | 25 mg/m² | Administered on Day 1 and Day 8 for 21 days in one cycle | IV |
| | Gemcitabine | 1000 mg/m² | Administered on Day 1 and Day 8 for 21 days in one cycle | IV |
| D-8 | Albumin-bound paclitaxel | 125 mg/m² | Administered on Day 1, Day 8, and Day 15 for 28 days in one cycle | IV |
| | Gemcitabine | 1000 mg/m² | Administered on Day 1, Day 8, and Day 15 for 28 days in one cycle | IV |

| | | | | |
|---|---|---|---|---|
| **IV: intravenously, Q2W: once every two weeks, Q3W: once every three weeks, QD: once a day In the table, Cohort "Dn" refers to that it is common to D-2 to D-8.** | | | | |

The trial treatment performed in each cohort is as follows.
- Cohort D-2: 360 mg/body of AB122 is administered by drip infusion over 60 minutes on Day 1. 80 mg/m² of cisplatin is administered intravenously on Day 1. 800 mg/m²/day of 5-fluorouracil is continuously administered intravenously from Day 1 to Day 5. Futibatinib is orally administered once a day on an empty stomach 1 hour or more before a meal or 2 hours or more after a meal.
- Cohort D-3: 360 mg/body of AB122 is administered by drip infusion over 60 minutes on Day 1. 1200 mg/body of AB154 is administered by drip infusion over 60 minutes on Day 1. Futibatinib is orally administered once a day on an empty stomach 1 hour or more before a meal or 2 hours or more after a meal.
- Cohort D-4: 360 mg/body of AB122 is administered by drip infusion over 60 minutes on Day 1. 100 mg/m² of carboplatin AUC 5 or cisplatin is administered intravenously on Day 1. 1000 mg/m²/day of 5-fluorouracil is continuously administered intravenously from Day 1 to Day 4. Futibatinib is orally administered once a day on an empty stomach 1 hour or more before a meal or 2 hours or more after a meal.
- Cohort D-5: 360 mg/body of AB122 is administered by drip infusion over 60 minutes on Day 1. 1200 mg/body of AB154 is administered by drip infusion over 60 minutes on Day 1. Futibatinib is orally administered once a day on an empty stomach 1 hour or more before a meal or 2 hours or more after a meal.
- Cohort D-6: 360 mg/body of AB122 is administered by drip infusion over 60 minutes on Day 1. 100 mg/m² of albumin-bound paclitaxel is administered intravenously on Day 1, Day 8, and Day 15, and carboplatin AUC 6 is administered intravenously on Day 1. Futibatinib is orally administered once a day on an empty stomach 1 hour or more before a meal or 2 hours or more after a meal.
- Cohort D-7: 360 mg/body of AB122 is administered by drip infusion over 60 minutes on Day 1. 25 mg/m² of cisplatin and 1000 mg/m² of gemcitabine are administered intravenously on Day 1 and Day 8. Futibatinib is orally administered once a day on an empty stomach 1 hour or more before a meal or 2 hours or more after a meal.
- Cohort D-8: 240 mg/body of AB122 is administered by drip infusion over 60 minutes on Day 1 and Day 15. 125 mg/m² of albumin-bound paclitaxel and 1000 mg/m² of gemcitabine are administered intravenously on Day 1, Day 8, and Day 15. Futibatinib is orally administered once a day on an empty stomach 1 hour or more before a meal or 2 hours or more after a meal.

Note that, when toxicity occurs at the start of the cycle or during the cycle, the dose can be changed stepwise according to the following table. The doses of AB122 and AB154 are unchanged during the administration period.

**Table 4**

| **Study drug name** | **Cohort** | **Dose level** |
|---|---|---|
| Futibatinib | Cohort D-n | Level 1: 20 mg QD |
| | | Level -1: 16g QD |
| | | Level -2: 12g QD |
| 5-Fluorouracil | Cohort D-2 | Level 1: 4000 mg/m²/cycle |
| | | Level -1: 3000 mg/m²/cyde |
| | | Level -2: 2000 mg/m²/cyde |
| Cisplatin | Cohort D-2 | Level 1: 80 mg/m² |
| | | Level -1: 60 mg/m² |
| | | Level -2: 40 mg/m² |
| Carboplatin | Cohort D-4 | Level 1: AUC 5 |
| | | Level -1: AUC 4 |
| | | Level -2: AUC 3 |
| Cisplatin | Cohort D-4 | Level 1: 100 mg/m² |
| | | Level -1: 80 mg/m² |
| | | Level -2: 64 mg/m² |
| 5-Fluorouracil | Cohort D-4 | Level 1: 4000 mg/m²/cycle |
| | | Level -1: 3200 mg/m²/cyde |
| | | Level -2: 2560 mg/m²/cyde |
| Carboplatin | Cohort D-6 | Level 1: AUC 6 |
| | | Level -1: AUC 5 |
| | | Level -2: AUC 4 |
| Albumin-bound paclitaxel | Cohort D-6 | Level 1: 100 mg/m² |
| | | Level -1: 75 mg/m² |
| | | Level -2: 50 mg/m² |
| Cisplatin | Cohort D-7 | Level 1: 25 mg/m² |
| | | Level -1: 18.75 mg/m² |
| | | Level -2: 12.5 mg/m² |
| Gemcitabine | Cohort D-7 | Level 1: 1000 mg/m² |
| | | Level -1: 800 mg/m² |
| Albumin-bound paclitaxel | Cohort D-8 | Level 1: 125 mg/m² |
| | | Level -1: 100 mg/m² |
| | | Level -2: 75 mg/m² |
| Gemcitabine | Cohort D-8 | Level 1: 1000 mg/m² |
| | | Level -1: 800 mg/m² |

| | | |
|---|---|---|
| **In the table, Cohort "D-n" refers to that t is common to D-2 to D-8.** | | |

### Phase 1b

Dose levels are determined based on the tolerability of each cohort in Phase 1a.

### Treatment Duration:

Cohort D-8: One cycle is defined as 28 days.
Cohort D-2 to D-7: One cycle is defined as 21 days.

The trial treatment is continued until any of the criteria for discontinuation of administration is met.

### Evaluation Criteria:

### Efficacy

RECIST (v1.1, 2009) is used for tumor assessment during the trial. Computed tomography (CT) scans are performed at baseline, every 4 weeks after registration, every 6 weeks after Week 12 and at the time of discontinuation.

### Safety

Standard safety monitoring and grading using NCI CTCAE v5.0 are performed.

### Statistical Procedure:

The analysis target population of this trial is defined for each phase and cohort, and the DLT evaluable case is defined for each cohort of Phase 1a.

**Table 5**

| Definition of Analysis Target Population in Each Phase and Cohort | |
|---|---|
| **Analysis target population** | **Definition** |
| Registration case | All patients registered in each cohort |
| Study drug administration case | All patients administered any study drug in each cohort among registration cases |
| DLT evaluable case | All patients except for the following cases among study drug administration cases in Phase 1a tolerability part |
| | - Patient receiving drug prohibited for combined use or therapy prohibited for combined use before DLT expression in Cycle 1 |
| | - Patient not administered AB122 or AB154 (only cohorts D-3 and D-5) on Day 1 in Cycle 1 or not completed to be administered study drug in Cycle 1 for reasons other than adverse events associated with treatment |
| | - Patient whose DLT is not recognized in Cycle 1 and the number of adm inistration days/total dose of futibatinib is less than 75% of the specified value, in which skip of administration due to adverse events not associated with study drug administration or other procedures with respect to administration are included as receiving of administration |
| | - Patient not receiving any inspection or observation specified in Cycle 1 |
| | - Patient withdrawing consent to trial participation or stopping administration of study drug for reasons other than adverse events associated with study drug in Cycle 1 |
| FAS | All patients receiving once or more evaluation of efficacy endpoint (any of primary endpoint and secondary endpoint) after administration of study drug among study drug administration cases |
| PK analysis target population | All patients whose AB122 concentration in serum or AB154 ooncentration in serum can be evaluated among study drug administration cases |
| ADA analysis target population | All patients whose ADA of AB122 or AB154 in serum can be evaluated among study drug administration cases |
| Predictive biomarker analysis target population | All patients whose data of predictive biomarker measured using tumor biopsy-derived tissue samples, preserved tumor tissue samples, or blood samples can be evaluated and evaluated data of predictive biomarker have been provided before registration |
| Pharmacodynamic biomarker analysis target population among study drug administration cases | All patients whose pharmacodynamic data measured using tumor biopsy-derived tissue samples can be evaluated among study drug administration cases |

| | |
|---|---|
| **ADA: anti-drug antibody, DLT: dose-limiting toxicity, FAS: maximum analysis target population, PK pharmacokinetics** | |

### Analysis on Primary Endpoint

### Phase 1a:

The occurrence ratio of DLT is calculated for each level of the DLT evaluable case. All DLTs are listed by patient.

### Phase 1b:

ORR analysis based on RECIST v1.1 is performed on the population obtained by merging FAS of Phase 1a and Phase 1b. Best overall response based on RECIST v1.1 is tabulated and ORR and 95% CI are estimated using the Clopper-Pearson method.

An outline of a clinical trial design is shown in Fig. 1.

### Results

For cohorts D-2, D-3, and D-7, the tolerability part resulted in no dose-limiting toxicity at the dose of Level 1 and confirmed tolerability.

Further expansion part is ongoing and intermediate results for cohort D-2 are shown in the table below. The ORR (objective response rate) and DCR (disease control rate) in patients in the tolerability part (N = 3, Level 1) and the expansion part (N = 38) were 58.5% and 92.7%, respectively. These values of ORR and DCR exceed the values (ORR (45.0%), DCR (79.4%)) in the combination therapy of pembrolizumab, 5-fluorouracil, and cisplatin, which is currently recommended as the primary treatment for radically unresectable advanced/recurrent esophageal cancer (Lancet. 2021; 398 (10302): 759-771.).

**Table 6**

| Summary of Demographics and Other Baseline Characteristics | | |
|---|---|---|
| | | Tolerability + Expansion(N=41) N (%) |
| Sex | | |
| | Male | 31 (75 6) |
| | Female | 10 (24 4) |

| Age (years) | | |
|---|---|---|
| | N | 41 |
| | Mean (S.D.) | 60.4 (11.0) |
| | Median | 61.0 |
| | IQR [Q1, Q3] | [53.0, 70.0) |
| | [Min, Max] | [33 , 80] |

| Age Category1 (years) | | |
|---|---|---|
| | <65 | 24 (58.5) |
| | >=65 | 17 (41.5) |

| Race | | |
|---|---|---|
| | American Indian or Alaska Native | 0 |
| | Asian | 41 (100.0) |
| | Black or African American | 0 |
| | Caucasian/White | 0 |
| | Native Hawaiian or Other Pacific | 0 |
| | Islander | |
| | Other | 0 |

| Ethnicity | | |
|---|---|---|
| | Japanese | 41 (100.0) |
| | Other | 0 |

| Height (cm) | | |
|---|---|---|
| | N | 41 |
| | Mean (S.D.) | 166.00 (7.48) |
| | Median | 166.20 |
| | IQR [QI, Q3] | [159.50 , 171.60] |
| | [Min, Max] | [148.5 , 181.1] |

| Weight (kg) | | |
|---|---|---|
| | N | 41 |
| | Mean (S.D.) | 58.03 (13.51) |
| | Median | 57.00 |
| | IQR [QI, Q3] | [46.10 , 65.50] |
| | [Min, Max] | [38.1 , 95.9] |

| BSA (m2) | | |
|---|---|---|
| | N | 41 |
| | Mean (S.D.) | 1.635 (0.198) |
| | Median | 1.640 |
| | IQR [Q1, Q3] | [1.440 , 1.770] |
| | [Min, Max] | [1.31 , 2.11] |

| PS | | |
|---|---|---|
| | 0 | 31 (75 6) |
| | 1 | 10 (24 4) |
| | 2 | 0 |
| | 3 | 0 |
| | 4 | 0 |

| | | |
|---|---|---|
| Analysis Set: Full Analysis Set | | |

### Best Overall Response (Investigator) (RECIST v1.1)

| | Tolerability + Expansion(N=41) N (%) |
|---|---|
| CR | 0 (0.0) |
| PR | 24 (58.5) |
| SD | 14 (34.1) |
| PD | 1 (2.4) |
| NE | 2 (4.9) |
| Objective Response Rate (CR+PR) | 24 (58.5) |
| 95%CI (%) | [42.1, 73.7] |
| One-sided P-Value* | 0.057 |
| Disease Control Rate (CR+PR+SD) | 38 (92.7) |
| 95%CI (%) | [80.1, 98.5] |

| | |
|---|---|
| * Binomial test under the threshold ORR as null | |

### Adverse Events by Each Grade

### Tolerability + Expansion (N=43)

| MedDRA(ver.27.0) | | Grade 1 | Grade 2 | Grade 3 | Grade 4 | Grade 5 | >=Grade 3 | Total | |
|---|---|---|---|---|---|---|---|---|---|
| Preferred Term | | N (%) | N (%) | N (%) | N (%) | N (%) | N (%) | N (%) | 95% CI |
| Any Events | | 1 (2.3) | 11 (25.6) | 27 (62.8) | 1 (23) | 3 (7.0) | 31 (72.1) | 43 (100.0) | [91.8, 100.0] |
| | Hyperphosphataemia | 5 (11.6) | 30 (69.8) | 0 (0.0) | 0 (0.0) | 0 (0.0) | 0 (0.0) | 35 (81.4) | [66.6, 91.6] |
| | Diarrhoea | 15 (34.9) | 12 (27.9) | 2 (4.7) | 0 (0.0) | 0 (0.0) | 2 (4.7) | 29 (67.4) | [51.5, 80.9] |
| | Nausea | 13 (302) | 12 (27.9) | 2 (4.7) | 0 (0.0) | 0 (0.0) | 2 (4.7) | 27 (62.8) | [46.7, 77.0] |
| | Decreased appetite | 9 (20.9) | 14 (32.6) | 4 (9.3) | 0 (0.0) | 0 (0.0) | 4 (9.3) | 27 (62.8) | [46.7, 77.0] |
| | Constipation | 16 (37.2) | 8 (18.6) | 0 (0.0) | 0 (0.0) | 0 (0.0) | 0 (0.0) | 24 (55.8) | [39.9, 70.9] |
| | Anaemia | 0 (0.0) | 10 (233) | 13 (30.2) | 0 (0.0) | 0 (0.0) | 13 (30.2) | 23 (53.5) | [37.7, 68.8] |
| | Stomatitis | 10 (23.3) | 10 (23.3) | 2 (4.7) | 0 (0.0) | 0 (0.0) | 2 (4 7) | 22 (51.2) | [35.5, 66.7] |
| | Neutrophil count decreased | 0 (0.0) | 3 (7.0) | 11 (25.6) | 2 (4.7) | 0 (0.0) | 13 (302) | 16 (37.2) | [23.0, 53.3] |
| | Alanine aminotransferase increased | 8 (18.6) | 3 (7.0) | 4 (9.3) | 0 (0.0) | 0 (0.0) | 4 (9.3) | 15 (34.9) | [21.0, 50.9] |
| | Palmar-plantar erythrodysacsthesia syndrome | 7 (16.3) | 6 (14.0) | 0 (0.0) | 0 (0.0) | 0 (0.0) | 0 (0.0) | 13 (30.2) | [17.2, 46.1] |
| | Alopecia | 7 (16.3) | 5 (11.6) | 0 (0.0) | 0 (0.0) | 0 (0.0) | 0 (0.0) | 12 (27.9) | [15.3, 43.7] |
| | Malaise | 6 (14.0) | 5 (11.6) | 0 (0.0) | 0 (0.0) | 0 (0.0) | 0 (0.0) | 11 (25.6) | [13.5, 41.2] |
| | Aspartate aminotransferase increased | 8 (18.6) | 2 (4.7) | 1 (23) | 0 (0.0) | 0 (0.0) | 1 (2.3) | 11 (25.6) | [13.5, 41.2] |
| | Blood creatinine increased | 9 (20.9) | 2 (4.7) | 0 (0.0) | 0 (0.0) | 0 (0.0) | 0 (0.0) | 11 (25.6) | [13.5, 41.2] |
| | White blood cell count decreased | 2 (4.7) | 7 (16.3) | 2 (4.7) | 0 (0.0) | 0 (0.0) | 2 (4.7) | 11 (25.6) | [13.5, 41.2] |
| | Dysgeusia | 9 (20.9) | 2 (4.7) | 0 (0.0) | 0 (0.0) | 0 (0.0) | 0 (0.0) | 11 (25.6) | [13.5, 41.2] |
| | Insomnia | 8 (18.6) | 3 (70) | 0 (0.0) | 0 (0.0) | 0 (0.0) | 0 (0.0) | 11 (25.6) | [13.5, 41.2] |
| | Hiccups | 5 (11.6) | 4 (9 3) | 0 (0.0) | 0 (0.0) | 0 (0.0) | 0 (0.0) | 9 (20.9) | [10.0, 36.0] |
| | Nail disorder | 5 (11.6) | 3 (7.0) | 0 (0.0) | 0 (0.0) | 0 (0.0) | 0 (0.0) | 8 (18.6) | [8.4, 33.4] |
| | Hyponatraemia | 0 (0.0) | 3 (7.0) | 4 (9.3) | 0 (0.0) | 0 (0.0) | 4 (9.3) | 7 (16.3) | [6.8, 30.7] |
| | Vomiting | 4 (9.3) | 1 (2.3) | 1 (2.3) | 0 (0.0) | 0 (0.0) | 1 (2.3) | 6 (14.0) | [5.3, 27.9] |
| | Platelet count decreased | 4 (9.3) | 2 (4.7) | 0 (0.0) | 0 (0.0) | 0 (0.0) | 0 (0.0) | 6 (14.0) | [5.3, 27.9] |
| | Weight increased | 4 (9.3) | 2 (4.7) | 0 (0.0) | 0 (0.0) | 0 (0.0) | 0 (0.0) | 6 (14.0) | [5.3, 27.9] |
| | Cheilitis | 0 (0.0) | 5 (11.6) | 0 (0.0) | 0 (0.0) | 0 (0.0) | 0 (0.0) | 5 (11.6) | [3.9, 25.1] |
| | Oedema peripheral | 5 (11.6) | 0 (0.0) | 0 (0.0) | 0 (0.0) | 0 (0.0) | 0 (0.0) | 5 (11.6) | [3.9, 25.1] |
| | Paronychia | 0 (0.0) | 5 (11.6) | 0 (0.0) | 0 (0.0) | 0 (0.0) | 0 (0.0) | 5 (11.6) | [3.9, 25.1] |
| | Phlebitis | 2 (4.7) | 3 (7.0) | 0 (0.0) | 0 (0.0) | 0 (0.0) | 0 (0.0) | 5 (11.6) | [3.9, 25.1] |

| | | | | | | | | | |
|---|---|---|---|---|---|---|---|---|---|
| Analysis Set: All-Treated Population If a patient is reported to have the same toxicity more than once, then that patient is only counted once for the summary of that toxicity, using the most severe intensity. | | | | | | | | | |

## Claims

1. An antitumor agent of any one of the following (i) to (iii) (provided that an antitumor agent comprising pembrolizumab as an active ingredient and an antitumor agent used so as to be administered in combination with pembrolizumab are excluded):
(i) the antitumor agent comprising, as an active ingredient, futibatinib or a pharmaceutically acceptable salt thereof, which is used so as to be administered to a cancer patient in combination with an immune checkpoint inhibitor and at least one or more other antitumor agents;
(ii) the antitumor agent comprising, as an active ingredient, an immune checkpoint inhibitor, which is used so as to be administered to a cancer patient in combination with futibatinib or a pharmaceutically acceptable salt thereof and at least one or more other antitumor agents; and
(iii) the antitumor agent comprising, as an active ingredient, at least one or more other antitumor agents, which is used so as to be administered to a cancer patient in combination with futibatinib or a pharmaceutically acceptable salt thereof and an immune checkpoint inhibitor.

2. The antitumor agent according to claim 1, wherein the immune checkpoint inhibitor is at least one selected from the group consisting of an anti-PD-1 antibody, an anti-PD-L1 antibody, and an anti-PD-L2 antibody.

3. The antitumor agent according to claim 1 or 2, wherein the immune checkpoint inhibitor is an anti-PD-1 antibody.

4. The antitumor agent according to claim 2 or 3, wherein the anti-PD-1 antibody is at least one selected from the group consisting of nivolumab, cemiplimab, spartalizumab, tislelizumab, BI754091, dostarlimab, sasanlimab, MGA-012, cetrelimab, AGEN-2034, zimberelimab, camrelizumab, budigalimab, and balstilimab.

5. The antitumor agent according to claim 4, wherein the anti-PD-1 antibody is zimberelimab.

6. The antitumor agent according to any one of claims 1 to 5, wherein the other antitumor agent is a chemotherapeutic agent.

7. The antitumor agent according to claim 6, wherein the chemotherapeutic agent is at least one selected from the group consisting of an antimetabolite, an alkaloid-based antitumor agent, a platinum preparation, an immune checkpoint inhibitor, a molecular targeting drug, an antitumor antibiotic, and an alkylating agent.

8. The antitumor agent according to claim 6, wherein the chemotherapeutic agent is at least one selected from the group consisting of an antimetabolite, an alkaloid-based antitumor agent, a platinum preparation, and an immune checkpoint inhibitor.

9. The antitumor agent according to claim 6, wherein the chemotherapeutic agent is at least one selected from the group consisting of fludarabine, cladribine, nelarabine, 5-fluorouracil, tegafur/gimeracil/oteracil potassium, tegafur/uracil, trifluridine/tipiracil hydrochloride, capecitabine, doxifluridine, 5-fluoro-2'-deoxyuridine, gemcitabine, cytarabine, pemetrexed, methotrexate, paclitaxel, albumin-bound paclitaxel, docetaxel, cabazitaxel, eribulin, irinotecan, nogitecan (topotecan), etoposide, teniposide, vinorelbine, vincristine, vinblastine, cisplatin, carboplatin, oxaliplatin, nedaplatin, domvanalimab, AB308, vibostolimab, ociperlimab, and tiragolumab.

10. The antitumor agent according to claim 6, wherein the chemotherapeutic agent is at least one selected from the group consisting of 5-fluorouracil, gemcitabine, albumin-bound paclitaxel, irinotecan, cisplatin, carboplatin, domvanalimab, and AB308.

11. The antitumor agent according to claim 6, wherein the chemotherapeutic agent is at least one selected from the group consisting of 5-fluorouracil, gemcitabine, albumin-bound paclitaxel, cisplatin, carboplatin, and domvanalimab.

12. The antitumor agent according to any one of claims 1 to 11, wherein the cancer is at least one selected from the group consisting of lung cancer, esophageal cancer, gastric cancer, duodenum cancer, liver cancer, hepatocellular cancer, biliary tract cancer, pancreatic cancer, large bowel cancer, breast cancer, uterine cancer, ovarian cancer, renal cancer, bladder cancer, prostate cancer, testicular tumor, thyroid cancer, bone or soft tissue tumor, leukemia, malignant lymphoma, multiple myeloma, head and neck cancer, brain tumor, mesothelioma, skin cancer, and cancer of unknown primary site.

13. The antitumor agent according to any one of claims 1 to 11, wherein the cancer is at least one selected from the group consisting of pancreatic cancer, lung cancer, esophageal cancer, biliary tract cancer, and head and neck cancer.

14. The antitumor agent according to any one of claims 1 to 13, wherein the cancer patient is a cancer patient who has not been previously treated for advanced cancer or who has been previously treated with one line of chemotherapy.

15. The antitumor agent according to any one of claims 1 to 14, wherein a 21-day or 28-day administration cycle is repeated once or twice or more.

16. The antitumor agent according to claim 15, wherein futibatinib is administered once a day on each day of the 21-day or 28-day administration cycle.

17. The antitumor agent according to claim 15, wherein zimberelimab is administered for one to two days in the 21-day or 28-day administration cycle.

18. The antitumor agent according to claim 15, wherein the other antitumor agent is administered for one to five days in the 21-day or 28-day administration cycle.

19. The antitumor agent according to claim 15, wherein the other antitumor agent is administered once on Day 1 in the 21-day or 28-day administration cycle.

20. The antitumor agent according to claim 15, wherein the other antitumor agent is administered once each on Day 1 and Day 8 in the 21-day or 28-day administration cycle.

21. The antitumor agent according to claim 15, wherein the other antitumor agent is administered once each on Day 1, Day 8, and Day 15 in the 21-day or 28-day administration cycle.

22. The antitumor agent according to claim 15, wherein the other antitumor agent is administered once each on Days 1, 2, 3, and 4 in the 21-day or 28-day administration cycle.

23. The antitumor agent according to claim 15, wherein the other antitumor agent is administered once each on Days 1, 2, 3, 4, and 5 in the 21-day or 28-day administration cycle.

24. The antitumor agent according to claim 16, wherein a dose of futibatinib is 8 mg/dose to 24 mg/dose.

25. The antitumor agent according to claim 16, wherein a dose of futibatinib is 12 mg/dose, 16 mg/dose, or 20 mg/dose.

26. The antitumor agent according to claim 16, wherein a dose of futibatinib is 20 mg/dose.

27. The antitumor agent according to claim 17, wherein a dose of zimberelimab is 240 mg/dose or 360 mg/dose.

28. The antitumor agent according to any one of claims 1 to 27, wherein the antitumor agent is an antitumor agent of any one of the following (i) to (iv):
(i) the antitumor agent comprising, as an active ingredient, futibatinib or a pharmaceutically acceptable salt thereof, which is used so as to be administered to a patient with esophageal cancer in combination with zimberelimab, cisplatin, and 5-fluorouracil;
(ii) the antitumor agent comprising, as an active ingredient, zimberelimab, which is used so as to be administered to a patient with esophageal cancer in combination with futibatinib or a pharmaceutically acceptable salt thereof, cisplatin, and 5-fluorouracil;
(iii) the antitumor agent comprising, as an active ingredient, cisplatin, which is used so as to be administered to a patient with esophageal cancer in combination with futibatinib or a pharmaceutically acceptable salt thereof, zimberelimab, and 5-fluorouracil; and
(iv) the antitumor agent comprising, as an active ingredient, 5-fluorouracil, which is used so as to be administered to a patient with esophageal cancer in combination with futibatinib or a pharmaceutically acceptable salt thereof, zimberelimab, and cisplatin.

29. The antitumor agent according to claim 28, wherein a 21-day administration cycle in which 12 mg/dose, 16 mg/dose, or 20 mg/dose of futibatinib is administered daily once a day, 360 mg/dose of zimberelimab is administered once, 80 mg/m²/dose of cisplatin is administered once, and 800 mg/m²/day of 5-fluorouracil is administered daily for 5 days is repeated once or twice or more.

30. The antitumor agent according to claim 28, wherein a 21-day administration cycle in which 12 mg/dose, 16 mg/dose, or 20 mg/dose of futibatinib is administered daily once a day, 360 mg/dose of zimberelimab is administered once on Day 1, 80 mg/m²/dose of cisplatin is administered once on Day 1, and 800 mg/m²/day of 5-fluorouracil is administered daily on Days 1 to 5 is repeated once or twice or more.

31. The antitumor agent according to any one of claims 28 to 30, wherein the cancer patient is a patient with esophageal cancer who has not been previously treated for advanced cancer.

32. The antitumor agent according to any one of claims 1 to 27, wherein the antitumor agent is an antitumor agent of any one of the following (i) to (iii):
(i) the antitumor agent comprising, as an active ingredient, futibatinib or a pharmaceutically acceptable salt thereof, which is used so as to be administered to a patient with esophageal cancer in combination with zimberelimab and domvanalimab;
(ii) the antitumor agent comprising, as an active ingredient, zimberelimab, which is used so as to be administered to a patient with esophageal cancer in combination with futibatinib or a pharmaceutically acceptable salt thereof, and domvanalimab; and
(iii) the antitumor agent comprising, as an active ingredient, domvanalimab, which is used so as to be administered to a patient with esophageal cancer in combination with futibatinib or a pharmaceutically acceptable salt thereof, and zimberelimab.

33. The antitumor agent according to claim 32, wherein a 21-day administration cycle in which 12 mg/dose, 16 mg/dose, or 20 mg/dose of futibatinib is administered daily once a day, 360 mg/dose of zimberelimab is administered once, and 1200 mg/dose of domvanalimab is administered once is repeated once or twice or more.

34. The antitumor agent according to claim 32, wherein a 21-day administration cycle in which 12 mg/dose, 16 mg/dose, or 20 mg/dose of futibatinib is administered daily once a day, 360 mg/dose of zimberelimab is administered once on Day 1, and 1200 mg/dose of domvanalimab is administered once on Day 1 is repeated once or twice or more.

35. The antitumor agent according to any one of claims 32 to 34, wherein the cancer patient is a patient with esophageal cancer who has not been previously treated for advanced cancer or who has been previously treated with one line of chemotherapy.

36. The antitumor agent according to any one of claims 1 to 27, wherein the antitumor agent is an antitumor agent of any one of the following (i) to (iv):
(i) the antitumor agent comprising, as an active ingredient, futibatinib or a pharmaceutically acceptable salt thereof, which is used so as to be administered to a patient with head and neck cancer in combination with zimberelimab, 5-fluorouracil, and carboplatin or cisplatin;
(ii) the antitumor agent comprising, as an active ingredient, zimberelimab, which is used so as to be administered to a patient with head and neck cancer in combination with futibatinib or a pharmaceutically acceptable salt thereof, 5-fluorouracil, and carboplatin or cisplatin;
(iii) the antitumor agent comprising, as an active ingredient, 5-fluorouracil, which is used so as to be administered to a patient with head and neck cancer in combination with futibatinib or a pharmaceutically acceptable salt thereof, zimberelimab, and carboplatin or cisplatin; and
(iv) the antitumor agent comprising, as an active ingredient, carboplatin or cisplatin, which is used so as to be administered to a patient with head and neck cancer in combination with futibatinib or a pharmaceutically acceptable salt thereof, zimberelimab, and 5-fluorouracil.

37. The antitumor agent according to claim 36, wherein a 21-day administration cycle in which 12 mg/dose, 16 mg/dose, or 20 mg/dose of futibatinib is administered daily once a day, 360 mg/dose of zimberelimab is administered once, AUC 5 carboplatin or 100 mg/m²/dose of cisplatin is administered once, and 1000 mg/m²/day of 5-fluorouracil is administered daily for 4 days is repeated once or twice or more.

38. The antitumor agent according to claim 36, wherein a 21-day administration cycle in which 12 mg/dose, 16 mg/dose, or 20 mg/dose of futibatinib is administered daily once a day, 360 mg/dose of zimberelimab is administered once on Day 1, AUC 5 carboplatin or 100 mg/m²/dose of cisplatin is administered once on Day 1, and 1000 mg/m²/day of 5-fluorouracil is administered daily on Days 1 to 4 is repeated once or twice or more.

39. The antitumor agent according to any one of claims 36 to 38, wherein the cancer patient is a patient with head and neck cancer who has not been previously treated for advanced cancer.

40. The antitumor agent according to any one of claims 1 to 27, wherein the antitumor agent is an antitumor agent of any one of the following (i) to (iii):
(i) the antitumor agent comprising, as an active ingredient, futibatinib or a pharmaceutically acceptable salt thereof, which is used so as to be administered to a patient with head and neck cancer in combination with zimberelimab and domvanalimab;
(ii) the antitumor agent comprising, as an active ingredient, zimberelimab, which is used so as to be administered to a patient with head and neck cancer in combination with futibatinib or a pharmaceutically acceptable salt thereof, and domvanalimab; and
(iii) the antitumor agent comprising, as an active ingredient, domvanalimab, which is used so as to be administered to a patient with head and neck cancer in combination with futibatinib or a pharmaceutically acceptable salt thereof, and zimberelimab.

41. The antitumor agent according to claim 40, wherein a 21-day administration cycle in which 12 mg/dose, 16 mg/dose, or 20 mg/dose of futibatinib is administered daily once a day, 360 mg/dose of zimberelimab is administered once, and 1200 mg/dose of domvanalimab is administered once is repeated once or twice or more.

42. The antitumor agent according to claim 40, wherein a 21-day administration cycle in which 12 mg/dose, 16 mg/dose, or 20 mg/dose of futibatinib is administered daily once a day, 360 mg/dose of zimberelimab is administered once on Day 1, and 1200 mg/dose of domvanalimab is administered once on Day 1 is repeated once or twice or more.

43. The antitumor agent according to any one of claims 40 to 42, wherein the cancer patient is a patient with head and neck cancer who has not been previously treated for advanced cancer.

44. The antitumor agent according to any one of claims 1 to 27, wherein the antitumor agent is an antitumor agent of any one of the following (i) to (iv):
(i) the antitumor agent comprising, as an active ingredient, futibatinib or a pharmaceutically acceptable salt thereof, which is used so as to be administered to a patient with non-small cell lung cancer in combination with zimberelimab, albumin-bound paclitaxel, and carboplatin;
(ii) the antitumor agent comprising, as an active ingredient, zimberelimab, which is used so as to be administered to a patient with non-small cell lung cancer in combination with futibatinib or a pharmaceutically acceptable salt thereof, albumin-bound paclitaxel, and carboplatin;
(iii) the antitumor agent comprising, as an active ingredient, albumin-bound paclitaxel, which is used so as to be administered to a patient with non-small cell lung cancer in combination with futibatinib or a pharmaceutically acceptable salt thereof, zimberelimab, and carboplatin; and
(iv) the antitumor agent comprising, as an active ingredient, carboplatin, which is used so as to be administered to a patient with non-small cell lung cancer in combination with futibatinib or a pharmaceutically acceptable salt thereof, zimberelimab, and albumin-bound paclitaxel.

45. The antitumor agent according to claim 44, wherein a 21-day administration cycle in which 12 mg/dose, 16 mg/dose, or 20 mg/dose of futibatinib is administered daily once a day, 360 mg/dose of zimberelimab is administered once, 100 mg/m²/dose of albumin-bound paclitaxel is administered three times, and AUC 6 carboplatin is administered once is repeated once or twice or more.

46. The antitumor agent according to claim 44, wherein a 21-day administration cycle in which 12 mg/dose, 16 mg/dose, or 20 mg/dose of futibatinib is administered daily once a day, 360 mg/dose of zimberelimab is administered on Day 1, 100 mg/m²/dose of albumin-bound paclitaxel is administered once on Day 1, Day 8, and Day 15, and AUC 6 carboplatin is administered on Day 1 is repeated once or twice or more.

47. The antitumor agent according to any one of claims 44 to 46, wherein the cancer patient is a patient with non-small cell lung cancer who has not been previously treated for advanced cancer.

48. The antitumor agent according to any one of claims 1 to 27, wherein the antitumor agent is an antitumor agent of any one of the following (i) to (iv):
(i) the antitumor agent comprising, as an active ingredient, futibatinib or a pharmaceutically acceptable salt thereof, which is used so as to be administered to a patient with biliary tract cancer in combination with zimberelimab, cisplatin, and gemcitabine;
(ii) the antitumor agent comprising, as an active ingredient, zimberelimab, which is used so as to be administered to a patient with biliary tract cancer in combination with futibatinib or a pharmaceutically acceptable salt thereof, cisplatin, and gemcitabine;
(iii) the antitumor agent comprising, as an active ingredient, gemcitabine, which is used so as to be administered to a patient with biliary tract cancer in combination with futibatinib or a pharmaceutically acceptable salt thereof, zimberelimab, and cisplatin; and
(iv) the antitumor agent comprising, as an active ingredient, cisplatin, which is used so as to be administered to a patient with biliary tract cancer in combination with futibatinib or a pharmaceutically acceptable salt thereof, zimberelimab, and gemcitabine.

49. The antitumor agent according to claim 48, wherein a 21-day administration cycle in which 12 mg/dose, 16 mg/dose, or 20 mg/dose of futibatinib is administered daily once a day, 360 mg/dose of zimberelimab is administered once, 25 mg/m²/dose of cisplatin is administered twice, and 1000 mg/m²/day of gemcitabine is administered twice is repeated once or twice or more.

50. The antitumor agent according to claim 48, wherein a 21-day administration cycle in which 12 mg/dose, 16 mg/dose, or 20 mg/dose of futibatinib is administered daily once a day, 360 mg/dose of zimberelimab is administered on Day 1, 25 mg/m²/dose of cisplatin is administered once on Day 1 and Day 8, and 1000 mg/m²/day of gemcitabine is administered once on Day 1 and Day 8 is repeated once or twice or more.

51. The antitumor agent according to any one of claims 48 to 50, wherein the cancer patient is a patient with biliary tract cancer who has not been previously treated for advanced cancer.

52. The antitumor agent according to any one of claims 1 to 27, wherein the antitumor agent is an antitumor agent of any one of the following (i) to (iv):
(i) the antitumor agent comprising, as an active ingredient, futibatinib or a pharmaceutically acceptable salt thereof, which is used so as to be administered to a patient with pancreatic cancer in combination with zimberelimab, albumin-bound paclitaxel, and gemcitabine;
(ii) the antitumor agent comprising, as an active ingredient, zimberelimab, which is used so as to be administered to a patient with pancreatic cancer in combination with futibatinib or a pharmaceutically acceptable salt thereof, albumin-bound paclitaxel, and gemcitabine;
(iii) the antitumor agent comprising, as an active ingredient, gemcitabine, which is used so as to be administered to a patient with pancreatic cancer in combination with futibatinib or a pharmaceutically acceptable salt thereof, zimberelimab, and albumin-bound paclitaxel; and
(iv) the antitumor agent comprising, as an active ingredient, albumin-bound paclitaxel, which is used so as to be administered to a patient with pancreatic cancer in combination with futibatinib or a pharmaceutically acceptable salt thereof, zimberelimab, and gemcitabine.

53. The antitumor agent according to claim 52, wherein a 28-day administration cycle in which 12 mg/dose, 16 mg/dose, or 20 mg/dose of futibatinib is administered daily once a day, 240 mg/dose of zimberelimab is administered twice, 125 mg/m²/dose of albumin-bound paclitaxel is administered three times, and 1000 mg/m²/dose of gemcitabine is administered three times is repeated once or twice or more.

54. The antitumor agent according to claim 52, wherein a 28-day administration cycle in which 12 mg/dose, 16 mg/dose, or 20 mg/dose of futibatinib is administered daily once a day, 240 mg/dose of zimberelimab is administered on Day 1 and Day 15, 125 mg/m²/dose of albumin-bound paclitaxel is administered once on Day 1, Day 8, and Day 15, and 1000 mg/m²/dose of gemcitabine is administered once on Day 1, Day 8, and Day 15 is repeated once or twice or more.

55. The antitumor agent according to any one of claims 52 to 54, wherein the cancer patient is a patient with pancreatic cancer who has not been previously treated for advanced cancer.
